# EUROPEAN PATENT APPLICATION

(11) **EP 1 454 914 A1**
(43) Date of publication of application: **08.09.2004**
(21) Application number: 02802375.2
(22) Date of filing: 29.10.2002
(51) Int. Cl.: C07K 7/04, C07K 14/28, C12N 15/12, A61K 38/04, A61K 38/17, A61K 39/00, A61K 39/395, A61K 48/00, A61P 35/00, C12P 21/02, C12Q 1/68, G01N 33/15, G01N 33/50, G01N 33/53, G01N 33/566

(54) **TUMOR ANTIGEN**

(30) Priority: 30.10.2001 JP 2001333219
(71) Applicant: Itoh, Kyogo, Miyaki-gun, Saga 841-0205 (JP)
(72) Inventor: ITOH, Kyogo, Miyaki-gun, Saga 841-0205 (JP); SHICHIJO, Shigeki, Kurume-shi, Fukuoka 830-0003 (JP)
(74) Representative: Krauss, Jan
(86) International application number: PCT/JP2002/011206
(87) International publication number: WO 2003/037917

(57) **Abstract**

A gene, which codes for a tumor antigen that is recognized by a cytotoxic T-lymphocyte (CTL) and/or induces a CTL in an HLA-A2-restricted manner, was isolated from a cDNA library of the human glioma cell strain KNS60 and identified. In addition, a peptide having the epitope of the tumor antigen was found, based on the tumor antigen coded by the gene obtained.

SEQUENCE LISTING

<110>  ITOH, Kyogo

<120>  Tumor antigen

<130>  GP02-1013PCT

<150>  JP P2001-333219
<151>  2001-10-30

<160>  23

<170>  PatentIn version 3.1

<210>  1
<211>  9
<212>  PRT
<213>  Artificial

<220>
<223>  Designed peptide recognized by HLA-A2-restricted cytotoxic T lymp
       hocytes

<400>  1

Thr Ile Met Ala Phe Arg Trp Val Thr
1               5


<210>  2
<211>  10
<212>  PRT
<213>  Artificial

<220>
<223>  Designed peptide recognized by HLA-A2-restricted cytotoxic T lymp
       hocytes

<400>  2

Ile Met Ser Arg Asp Leu Val Pro Arg Ile
1               5                   10


<210>  3
<211>  9
<212>  PRT
<213>  Artificial sequence

<220>
<223>  Designed peptide recognized by HLA-A2-restricted cytotoxic T lymp
       hocytes

<400>  3

Asn Leu Leu Lys Val Asn Ile His Ile
1               5


<210>  4
<211>  10
<212>  PRT

<213>  Artificial

<220>
<223>  Designed peptide recognized by HLA-A2-restricted cytotoxic T lymp
      hocytes

<400>  4

Phe Leu Pro His Phe Gln Ala Leu His Val
1               5                   10


<210>  5
<211>  9
<212>  PRT
<213>  Artificial

<220>
<223>  Designed peptide recognized by HLA-A2-restricted cytotoxic T lymp
      hocytes

<400>  5

Ala Leu His Val Val Val Ile Gly Leu
1               5


<210>  6
<211>  9
<212>  PRT
<213>  Artificial

<220>
<223>  Designed peptide recognized by HLA-A2-restricted cytotoxic T lymp
      hocytes

<400>  6

Gly Ile Thr Phe Gln Val Trp Asp Val
1               5


<210>  7
<211>  9
<212>  PRT
<213>  Artificial

<220>
<223>  Designed peptide recognized by HLA-A2-restricted cytotoxic T lymp
      hocytes

<400>  7

Cys Leu Gly Glu Glu Val Leu Glu Thr
1               5


<210>  8
<211>  9
<212>  PRT
<213>  Artificial

<220>

<223>  Designed peptide recognized by HLA-A2-restricted cytotoxic T lymp
      hocytes

<400>  8

Ile Ile Ile Gly Phe Phe Cys Tyr Thr
1               5


<210>  9
<211>  10
<212>  PRT
<213>  Artificial

<220>
<223>  Designed peptide recognized by HLA-A2-restricted cytotoxic T lymp
      hocytes

<400>  9

Gly Ile His Leu Ala Cys Phe Val Glu Val
1               5                   10


<210>  10
<211>  9
<212>  PRT
<213>  Artificial

<220>
<223>  Designed peptide recognized by HLA-A2-restricted cytotoxic T lymp
      hocytes

<400>  10

Ile Leu Trp Lys Glu Lys Asn Ser Ala
1               5


<210>  11
<211>  10
<212>  PRT
<213>  Artificial

<220>
<223>  Designed peptide recognized by HLA-A2-restricted cytotoxic T lymp
      hocytes

<400>  11

Asn Leu Val Ser Leu Phe Ser Arg Tyr Val
1               5                   10


<210>  12
<211>  10
<212>  PRT
<213>  Artificial

<220>
<223>  Designed peptide recognized by HLA-A2-restricted cytotoxic T lymp
      hocytes

<400>  12

Asn Gln Trp Thr Glu Val Met Phe Met Ala
1               5                   10


<210>  13
<211>  10
<212>  PRT
<213>  Artificial

<220>
<223>  Designed peptide recognized by HLA-A2-restricted cytotoxic T lymp
       hocytes

<400>  13

Val Met Phe Met Ala Thr Arg Glu Leu Leu
1               5                   10


<210>  14
<211>  204
<212>  PRT
<213>  Homo sapiens

<400>  14

Met Leu Ala Leu Ser Leu Glu Asp Glu His Leu Leu Tyr Gly Asp Ile
1               5                   10                  15


Ile Arg Gln Asp Phe Leu Asp Thr Tyr Asn Asn Leu Thr Leu Lys Thr
            20                  25                  30


Ile Met Ala Phe Arg Trp Val Thr Glu Phe Cys Pro Asn Ala Lys Tyr
        35                  40                  45


Val Met Lys Thr Asp Thr Asp Val Phe Ile Asn Thr Gly Asn Leu Val
    50                  55                  60


Lys Tyr Leu Leu Asn Leu Asn His Ser Glu Lys Phe Phe Thr Gly Tyr
65                  70                  75                  80


Pro Leu Ile Asp Asn Tyr Ser Tyr Arg Gly Phe Tyr Gln Lys Thr His
                85                  90                  95


Ile Ser Tyr Gln Glu Tyr Pro Phe Lys Val Phe Pro Pro Tyr Cys Ser
            100                 105                 110


Gly Leu Gly Tyr Ile Met Ser Arg Asp Leu Val Pro Arg Ile Tyr Glu
        115                 120                 125


Met Met Gly His Val Lys Pro Ile Lys Phe Glu Asp Val Tyr Val Gly
    130                 135                 140

Ile Cys Leu Asn Leu Leu Lys Val Asn Ile His Ile Pro Glu Asp Thr
145                 150                 155                 160

Asn Leu Phe Phe Leu Tyr Arg Ile His Leu Asp Val Cys Gln Leu Arg
                165                 170                 175

Arg Val Ile Ala Ala His Gly Phe Ser Ser Lys Glu Ile Ile Thr Phe
            180                 185                 190

Trp Gln Val Met Leu Arg Asn Thr Thr Cys His Tyr
        195                 200

<210>  15
<211>  201
<212>  PRT
<213>  Homo sapiens

<400>  15

Met Gly Asn His Leu Thr Glu Met Ala Pro Thr Ala Ser Ser Phe Leu
1               5                   10                  15

Pro His Phe Gln Ala Leu His Val Val Val Ile Gly Leu Asp Ser Ala
            20                  25                  30

Gly Lys Thr Ser Leu Leu Tyr Arg Leu Lys Phe Lys Glu Phe Val Gln
        35                  40                  45

Ser Val Pro Thr Lys Gly Phe Asn Thr Glu Lys Ile Arg Val Pro Leu
    50                  55                  60

Gly Gly Ser Arg Gly Ile Thr Phe Gln Val Trp Asp Val Gly Gly Gln
65                  70                  75                  80

Glu Lys Leu Arg Pro Leu Trp Arg Ser Tyr Thr Arg Arg Thr Asp Gly
                85                  90                  95

Leu Val Phe Val Val Asp Ala Ala Glu Ala Glu Arg Leu Glu Glu Ala
            100                 105                 110

Lys Val Glu Leu His Arg Ile Ser Arg Ala Ser Asp Asn Gln Gly Val
        115                 120                 125

Pro Val Leu Val Leu Ala Asn Lys Gln Asp Gln Pro Gly Ala Leu Ser
    130                 135                 140

Ala Ala Glu Val Glu Lys Arg Leu Ala Val Arg Glu Leu Ala Ala Ala
145                 150                 155                 160

Thr Leu Thr His Val Gln Gly Cys Ser Ala Val Asp Gly Leu Gly Leu
                165                 170                 175

Gln Gln Gly Leu Glu Arg Leu Tyr Glu Met Ile Leu Lys Arg Lys Lys
            180                 185                 190

Ala Ala Arg Gly Gly Lys Lys Arg Arg
        195                 200

<210>  16
<211>  73
<212>  PRT
<213>  Homo sapiens

<400>  16

Met Cys Leu Gly Glu Glu Val Leu Glu Thr His Pro Thr His Leu Glu
1               5                   10                  15

Ser His Gly Ile His Leu Ala Cys Phe Val Glu Val Val Gln Pro Asn
            20                  25                  30

Gly Gln Tyr Leu Lys Ser Thr Val Asn Leu His Thr Ile Ile Ile Gly
        35                  40                  45

Phe Phe Cys Tyr Thr Ser Ala Ser Gly Lys Gly Val Ser Leu Met Gln
    50                  55                  60

Ala Glu Lys Phe Arg Asp Tyr Lys Val
65                  70

<210>  17
<211>  111
<212>  PRT
<213>  Homo sapiens

<400>  17

Met His Thr Arg Leu Pro Leu Gln Val Asn Ser Lys Ala Ala Cys Lys
1               5                   10                  15

Val Pro Pro Phe Leu Gln Thr Leu Trp Val Val Ser Leu Gly Leu Asn
            20                  25                  30

Lys Trp Pro Asn Ile Cys Met Leu Thr Ala Ser Ser Ile Pro His Ala
        35                  40                  45

Ser Phe Val Ile Leu Trp Lys Glu Lys Asn Ser Ala Ile Phe Arg Glu
    50                  55                  60

Thr Ile Tyr Ser Tyr Leu Asn Met Glu Ala Phe Thr Arg Asn Ser Lys
65                  70                  75                  80

Tyr Ile Ser Phe His Leu Tyr Asn Glu Lys Lys Asp Glu Lys Ala Arg
                85                  90                  95

Leu Leu Leu Gln Leu Thr Leu Ser Ala Leu Pro Phe Gly Phe Met
            100                 105                 110

<210>  18
<211>  111
<212>  PRT
<213>  Homo sapiens

<400>  18

Met Asn Leu Pro Gln Ser Ala Ala Thr Pro Ala Leu Ser Asn Pro His
1               5                   10                  15

Leu Thr Ala Val Leu Gln Asn Lys Phe Gly Leu Ser Leu Leu Leu Ile
            20                  25                  30

Leu Leu Ser Arg Gly Glu Asp Leu Gln Ser Ser Asp Pro Ala Thr Glu
        35                  40                  45

Ser Thr Gln Asn Asn Gln Trp Thr Glu Val Met Phe Met Ala Thr Arg
    50                  55                  60

Glu Leu Leu Arg Ile Pro Gln Ala Ala Leu Ala Lys Pro Ile Ser Ile
65                  70                  75                  80

Pro Thr Asn Leu Val Ser Leu Phe Ser Arg Tyr Val Asp Arg Gln Lys
                85                  90                  95

Leu Asn Leu Leu Glu Thr Lys Leu Gln Leu Val Gln Gly Ile Arg
            100                 105                 110

<210>  19
<211>  2292
<212>  DNA
<213>  Homo sapiens

<400>  19
atcaaaatcc atttctggtc attctggtga cctcccaccc ttcagatgtg aaagccaggc      60

aggccattag agttacttgg ggtgaaaaaa agtcttggtg gggatatgag gttcttacat      120

ttttcttatt aggccaagag gctgaaaagg aagacaaaat gttggcattg tccttagagg      180

atgaacacct tctttatggt gacataatcc gacaagattt tttagacaca tataataacc      240

tgaccttgaa aaccattatg gcattcaggt gggtaactga gttttgcccc aatgccaagt      300

acgtaatgaa gacagacact gatgttttca tcaatactgg caatttagtg aagtatcttt 360

taaacctaaa ccactcagag aagtttttca caggttatcc tctaattgat aattattcct 420

atagaggatt ttaccaaaaa acccatattt cttaccagga gtatcctttc aaggtgttcc 480

ctccatactg cagtgggttg ggttatataa tgtccagaga tttggtgcca aggatctatg 540

aaatgatggg tcacgtaaaa cccatcaagt ttgaagatgt ttatgtcggg atctgtttga 600

atttattaaa agtgaacatt catattccag aagacacaaa tcttttcttt ctatatagaa 660

tccatttgga tgtctgtcaa ctgagacgtg tgattgcagc ccatggcttt tcttccaagg 720

agatcatcac tttttggcag gtcatgctaa ggaacaccac atgccattat taacttcaca 780

ttctacaaaa agcctagaag gacaggatac tttgtggaaa gtgttaaata aagtaggtac 840

tgtggaaaat tcatggggag gtcagtgtgc tggcttacac tgaactgaaa ctcatgaaaa 900

acccagactg gagactggag ggttacactt gtgatttatt agtcaggccc ttcaaagatg 960

atatgtggag gaattaaata taaaggaatt ggaggttttt gctaaagaaa ttaataggac 1020

caaacaattt ggacatgtca ttctgtagac tagaatttct taaaagggtg ttactgagtt 1080

ataagctcac taggctgtaa aaacaaaaca atgtagagtt ttatttattg aacaatgtag 1140

tcacttgaag gttttgtgta tatcttatgt ggattaccaa tttaaaaata tatgtagttc 1200

tgtgtcaaaa aacttcttca ctgaagttat actgaacaaa attttacctg tttttggtca 1260

tttataaagt acttcaagat gttgcagtat ttcacagtta ttattattta aaattacttc 1320

aactttgtgt ttttaaatgt tttgacgatt tcaatacaag ataaaaagga tagtgaatca 1380

ttctttacat gcaaacattt tccagttact taactgatca gtttattatt gatacatcac 1440

tccattaatg taaagtcata ggtcattatt gcatatcagt aatctcttgg actttgttaa 1500

atattttact gtggtaatat agagaagaat taaagcaaga aaatctgaag tattgtcttg 1560

tttttaaaaa atacagttcc tagtgttttt agaagtcact taatttgtct catttttcca 1620

cctggaaatt aggaataatg tagaatgcaa ggcagtaatt tccttttgga aaggactctg 1680

aaggcagaaa agaagggaga gaacctcatg ggcagaatat tataaaaaga gtgtcatatt 1740

ccagcatttg aattggaaag agaagagtga agatccaagt tgcattatta atctgccctg 1800

tgttttttcc ttttaacaat cagtttgagc tgctgctgtt atgagtttct catcaagatg 1860

aaagccctaa tatgtaaagt caaatccgat ttaaattttg tgcttttata gaaagaaatt 1920

tcttcataga cgtggtgata tatcatttgt tggacctgct aatagtaggt caaaggggag 1980

cactccttgc cccctgttcc tgggtttatg cagttttctt tttagagttt atatagggca 2040

agtggttctt tttctctgaa ttacaggatg gaaaaaggtc atatcctttg tcaggaaata 2100

taaacttgaa agtatgtagt cagctcttgt aatactcata tttatgattg tcctatatga 2160

aaaacaactt cagttaaaac tataatgtgt gattctgtat aacaaggtga tgtctgtttc 2220

ccagggctca gacctaatcc agttataata aaatcaatta aatgaaatat tctatagaaa 2280

aaaaaaaaaa aa 2292

<210> 20
<211> 1392
<212> DNA
<213> Homo sapiens

<400> 20
ggcgcggtgg gtgtctgcgg gggtctcgcg gggcggctgc ggtgtttcac cgggaaaggc 60

tcgaggagag cgcggctcac gagagataac ccagctgtgc tccctggaac cttcaatttc 120

aaggcctccc tgcctctact aggcgcctta gctcactatg gggaaccact tgactgagat 180

ggcgcccact gcctcctcct tcttgcccca cttccaagcc ctgcatgtcg tggtcattgg 240

gctggactct gctggaaaga cctccctcct ttaccgcctc aagttcaagg agtttgtcca 300

gagtgtcccc accaaaggct tcaacaccga gaagatccgg gtgcccctcg ggggatcgcg 360

tggcatcacc ttccaagtgt gggacgtcgg ggggcaggag aagctgcgac cactgtggcg 420

ctcttatacc cgccggacag acggtctagt gtttgtggtg gacgctgcgg aggctgagcg 480

gctggaggaa gccaaggtgg agttgcaccg aatcagccgg gcctcggaca accagggcgt 540

gccagtgctg gtgctggcca acaagcagga ccagcccggg gcactgagcg ctgctgaggt 600

ggagaagagg ctggcagtcc gagagctagc agccgccact ctcactcatg tgcaaggctg 660

cagcgctgtg gacggtctgg gcctgcagca gggccttgag cgcctctatg agatgatcct 720

caagaggaag aaggcagctc ggggtggcaa gaagagacgg tgacccaagc cccccctccc 780

tttcctccca cctagtaggg gtctgcacac ttggacagca gggtgggacc agcctgtgac 840

ctctcagtca gactggggtg caggacctgt ccacctcaat gaaggagaga ggagcatggg 900

gtgtcccgtt ttggtgccac actggggtgg ggatgggaga tgggatgtct ttgcatatct 960

ctctcatcct ctctggagaa gtgggcgctg caggactgtg gagacgtaaa tgtaaactgt 1020

gactctacct cgaccctgtt tcttattttt cttctctggc taaaaatttt taattggatg 1080

tgtttggggg cggggggatg gaagtgactt ggagaatgtg tttgggatga aataactatt 1140

tccccttcct ctgtccccca actggggagt ctccccaggc tgcttttcta ggaataccag 1200

tcacatagtt tttatttttg tgtctgtgaa agtgccaaga acccctcccc acatttgtag 1260

atccatgacc ctttttataa gctgtgtgtg tcctctgtat tattgttatt aactattttt 1320

tagcatttgc ctgtaagtta ttaaagactg ataactgtag ctcttaaaaa aaaaaaaaaa 1380

aaaaaaaaaa aa 1392

<210> 21
<211> 701

<212>  DNA
<213>  Homo sapiens

<400>  21
ccggcaaatg gttgagtcaa acccagtata gcaatgggat gtgtctgggc gaggaggtcc      60

ttgaaactca tcctactcac ttagaatctc atggaattca cctggcctgc tttgtagaag     120

ttgttcagcc caatggccaa tacttaaagt ctactgtgaa cctacacact attatcattg     180

ggtttttctg ctatacctca gcatcaggta agggagtatc tctgatgcaa gcggagaaat     240

tcagggatta caaggtatga actgagtggt tgtgaaatgt agaaggaaaa acacaggctt     300

taaagtcaga gaagtccaag ttgaaatgcc agttctgcca cctctcagcc acctaacttt     360

tgagagtctc actttataaa atgggaacaa tgattgcctt aaagggtggt tgtcaaggtt     420

aacaaactaa tgtttataaa atgcattgta gcaaagctta caatgcattt tataaacatt     480

tataaaagag ttacctatct tacctgttat cttctgctgt ccttattccc agcaagggtt     540

tggcaaatta caaaataagg atgatgaaaa atgctgaaat ccagttttgt caaattaaaa     600

gcagtttgtt tttctcatat gtcatctgct gtttccggag taaaaatatt cagtatcact     660

gaaaaaaaaa aaaaaaaaaa aaaaaaaaaa aaaaaaaaaa a                         701

<210>  22
<211>  1848
<212>  DNA
<213>  Homo sapiens

<400>  22
cagcttgatg ttagatggga ttctgtgacc gtttgtatac tgacaggtcc ttccagtctc      60

cctccacagt cccacaagcg gaagtattct acctgattcc agagttcatt ccaacatgca     120

gtagaatgag gtgaatgaag aaccaagtgt gggttttgtt ttcacagtgc acttggtcaa     180

tctagtttct ctgagtagtg ggctgtattc taagaaaagc tttcacgtag acgtcaaagt     240

ctgcaaaaga gatgagttag ccactttaca aaaacctgct ctgctttaca aaagatgcac     300

accaggcttc ctttacaagt gaactccaag gctgcttgta aagtaccacc cttcctgcag     360

actttgtggg tcgtgtcact tggcctaaac aagtggccta acatatgcat gctcacagct     420

agtagcatcc ctcatgcgtc ctttgtcata ctctggaaag aaaaaaactc tgctatcttc     480

agagaaacca tctattcata cttaaatatg gaagccttta caaggaactc aaaatacatt     540

tcatttcatt tgtacaatga gaaaaaagat gaaaaggcaa ggttgttatt gcagttgaca     600

ttgtcagccc tgccttttgg ctttatgtaa tttaaaatag aaagggggaa ggaggacaag     660

acagaagctc aactttagaa accatggaca tcacctccct ctttgagagg agctgcttgt     720

gaaccaggct ccagtcttcc tgtggcctct cacagtaacc ctgccccttt cttacctcac     780

aggggcaaga gggactctaa agaggcgtaa agcaatagag gcacctgcag ccctccttcc     840

tcagcaggaa atcaatgtgg agccccagag ggacatttta tctcagagag agcatgctgg 900

ccacaggacc cagtgcagcc ctgcttcctg caccgtgggt ctttcctgat agctgcaggc 960

ttgagattgc agcctcagga taggacacgt tcagatgcct tccagactac acacactatg 1020

cttagattcg agagtctttc ctgaccttaa aatttgtccc ttgcttactt tcacactgca 1080

gaagatttat atatatattc atcattattt ttgcagcagt acaagtggaa acttcaaaaa 1140

aatcattccc tttcatgtcc tggtcttttt aagtcttgat atgtaaccat tttcataaat 1200

atccaaacag agattgtcat tccccttaaa aacagaaaca aaacacaaaa ctagtgcttg 1260

tgaaaacaca cagcgcaaag acaattatcc tattgcatta aaaaaaaaaa tcagtctgcc 1320

tagagacatt tcattggctg cattgtctta attccctaac gaaactgcat caaaatgtca 1380

gttgtaattt agcctctgat cagctgttaa atattattta aatatttaca ggtcctgtgg 1440

ggtatctccc attacccatg tcgccctcaa attacataag cgatcataaa ataagaagaa 1500

aaaaaaaggt ctttagtaga tcacagcaga gttaccactg ataagagtgt tggctttgtt 1560

cagttgtttt taaattacca gccgtcaagc acagtagccc ctaaaaccaa ttttgagcaa 1620

ggataaattc aactccctca tctgagaacc ttgactttcc agttgccttg aagtattggg 1680

aatagtaact gtacattttc aatttaatta tttggtattt tatttgactg tcttggccat 1740

gtcattttat ggaagcattt ttaaatagca ctttttgtta gtggtgtgga atttctgcgg 1800

ttattttttt tttttttttg caacagatga agcaaaaaaa aaaaaaaa 1848

<210> 23
<211> 2039
<212> DNA
<213> Homo sapiens

<400> 23
cagccttttg cgacagctaa tgaacctacc tcaaagtgca gctacaccag cactctccaa 60

tcctcacctc actgctgtgc tccagaacaa gtttggcctg tcactgctcc tcatcctcct 120

gagccgtggt gaagacctac agagttcaga ccctgctaca gaatcaacac aaaataatca 180

gtggacggag gtgatgttca tggcaacacg agaacttctg cggattcccc aagcagccct 240

ggccaagcca atctctatac ctacaaacct agtgtccctc ttttctcgct atgttgaccg 300

gcagaaactg aacttgctgg agacaaaact gcagctagtt caggggatac gataaaagat 360

ctccaaatgt gtcctgtacc tccttttggc tgccacctgc actgctgcca tcaccaatgg 420

agtgttttta atgagggaag gaaggtagct ttttccccaa agcaaagtct tgtgggatcg 480

attcctgttt acaggggttg tctctctaaa tgtcagatat ttccccactg ctctatgaaa 540

tttggctggg tgatacttct gctggtttct ttaccttctg tgttacagtt ctgcatgtcc 600

tacttttact cagttctggt ttgcattttc tttgccctag agacacaagt gtaatctctc 660

cctttatccc tccactactc cacctcagag tagattgtag cctgccaaag gattccttcc 720

ctcatcctat tgaagttgtt ttttcattgc cccatattaa tatgactata gaagagccaa 780

ttaagtagaa atcaagatat acacacacac atagatacac acacacacac cccatacatg 840

tatttatgtg gtcttcagag ggtccttaaa gaatgaattt tagattgaaa aatatttagt 900

tgtctcatta cctcttctaa acacaaacca gctgatgtat tttaatctgt ttctgttcta 960

tcttgtaatt aatttggtgg gttctacttg ttttaacata aataaagagt atgcagcacg 1020

tttaataaaa tcagaactct taattggctt atgcccaggt ctaggctgag aagtcctttt 1080

tcttcttccc acctttattt ccttagtttc tgtccacctt aatcgaaaca acacatggtt 1140

atgtcttttt cctgctacaa ctacagggta cttgagcctt tcccctcaag tgcattcgaa 1200

gtcacccagg atgatcctca ctagtagcct gctttggcag tgtggctttt tgcacacttg 1260

ccctgtcttc ctgagactac ttcagtaagc catgcttcct tcttccccac ttttatttgg 1320

tgtcatgaat agaaacttca aatgtaacca tggaagctaa gtttggcctg ctttgctttt 1380

tagtctccac accatgggca gaactgctgt ctttactact tcatctcacc caagtcccgt 1440

tcccaggcag ccaggggcct gggtttgaat aattgcaggg ccagcctgcc atgatctttc 1500

tcacttactc ctctcccatt cagcaatcaa ccagactaag gagttttgat ccctagtgat 1560

tacagccctg aagaaaatta aatctgaatt aattttacat ggccttcgtg atctttctgc 1620

tgttcttact ttttcgaatg tagttggggg gtgggaggga caggttatgg tatttaaaga 1680

gaataaacat tttgcacata catgtattgt acaacagtaa gatcctctgt taaaaccagc 1740

tgtcctgttc tccatctcca tttcttccca tgctgtaacc ccaggctcca ccagctgttc 1800

cccagtgatg ttacctagct tccctctacc gttgtctact gaccatttcc actacatgcc 1860

tttcctacct tcccttcaca accaatcaag tgaatacttg attattatct cttccttact 1920

gtgctttatc tttttgtttt ggattggttc taattaatga aaataaaagt ttctaaattt 1980

acatttttat agggtattgt aaataaaaac aaattgtata cttaaaaaaa aaaaaaaaa 2039

## Description

### FIELD OF THE INVENTION

The present invention relates to a tumor antigen, more specifically, it relates to a peptide or a polypeptide that is recognized by tumor-specific cytotoxic T-lymphocytes; a polynucleotide that codes for the peptide or the polypeptide, or a polynucleotide that is the complementary strand thereof; a recombinant vector that comprises the polynucleotide; a transformant that comprises the recombinant vector; an antibody that is directed against the peptide or the polypeptide; a compound that interacts with the peptide, the polypeptide, or the polynucleotide; a medicament containing the peptide and/or the polypeptide; a preventive agent and/or a therapeutic agent for multiple sclerosis containing the peptide and/or the polypeptide; a cancer vaccine or a cytotoxic T-lymphocyte inducing agent that contains the peptide and/or the polypeptide; a pharmaceutical composition that contains one or more species thereof; a method for preparing the peptide or the polypeptide; a method for identifying the compound that interacts with the peptide, the polypeptide, or the polynucleotide; a method for inducing cytotoxic T-lymphocytes by using the peptide or the polypeptide; a method for measuring the peptide, the polypeptide, or the polynucleotide; as well as a reagent kit for use in the identification method or the measurement method.

### BACKGROUND OF THE INVENTION

In eliminating cancer *in vivo*, the immune system, and in particular, cytotoxic T-lymphocytes involved in cell-mediated immunity, play an important role. For instance, infiltration of a cytotoxic T-lymphocyte that demonstrates cytotoxicity against tumor cells has been observed at a tumor site in cancer patients (Non-Patent Reference 1). The target molecule of such tumor-specific cytotoxic T-lymphocytes, or so-called tumor antigens, was discovered for the first time in melanoma. The tumor antigen that is generated in a tumor cell is degraded in the cell into a peptide that consists of 8 to 11 amino acids, or a so-called tumor antigen peptide, which binds to a molecule of human leukocyte antigen (hereinafter abbreviated as HLA) which is a major histocompatibility complex (hereinafter abbreviated as MHC), and is presented on the surface of the tumor cell. The cytotoxic T-lymphocyte recognizes the complex comprising this HLA molecule and the tumor antigen peptide, releases a soluble factor, for example, a cytokine, such as interferon-γ, and then lyses the tumor cell. That is to say, the cytotoxic T-lymphocyte demonstrates a cytotoxic activity by recognizing tumor cells in an HLA-restricted manner.

HLA is a cell membrane antigen that is expressed in almost all eukaryotic cells. HLA is broadly divided into class I antigens and class II antigens; HLA recognized together with the antigen peptide by cytotoxic T-lymphocytes is a class I antigen. HLA class I antigens are further classified into HLA-A, HLA-B, HLA-C, and the like; and in humans, different eukaryotic cells have different quantities of HLA-A, HLA-B, and HLA-C. In addition, a genetic polymorphism therefor has been reported. For instance, for HLA-A there are such polymorphisms as A1, A2, A24, and A26; for HLA-B there are such polymorphisms as B8, B27, and B46; and for HLA-C there are such polymorphisms as Cw3 and Cw6. Thus, the types of HLA that each individual possesses are not necessarily identical. The HLA-A2 allele, which is one of polymorphism of the HLA-A sub-region, is found in approximately 40% of Japanese, approximately 53% of Chinese, approximately 49% of North American Caucasians, approximately 38% of South American Caucasians, and approximately 23% of African Blacks.

When the cytotoxic T-lymphocyte recognizes a complex of an HLA class I antigen and a tumor antigen peptide, it also recognizes the HLA type thereof. In addition, a motif (regular arrangement), which is different depending on the HLA type, is known to exist in the amino acid sequence of the tumor antigen peptide that binds to the HLA molecule. Since a peptide that binds to the HLA molecule differs depending on the type of HLA, it is necessary to select a peptide that binds to HLA of each type in order to induce and/or activate an antigen-specific cytotoxic T-lymphocyte using a tumor antigen peptide.

In recent years, molecules that are involved in specific immunity, such as tumor rejection antigen genes and T-cell antigen receptors (T-cell receptors), have been identified in a variety of cancers, for instance, in melanoma, esophageal cancer, and the like, and specific immunotherapies using peptides are being examined in advanced cancers and metastatic cancers (Non-Patent References 2, 3, 4, 5, 6, and 7).

Currently, in Europe and America, cancer vaccine therapies are being developed, wherein cytotoxic T-lymphocytes inside the body of a cancer patient are activated by administration of a tumor antigen, and results from clinical studies have been reported for melanoma specific tumor antigens. For example, as a result of subcutaneously administrating melanoma antigen gp100 peptide to melanoma patients and intravascularly administrating interleukin-2, reduction of tumor was observed in 42% of the patients (Non-Patent Reference 8). Thus, an effective cancer therapy effect can be expected from a tumor antigen, when it is used as a cancer vaccine.

However, when the diversity of cancers is considered, it is impossible to treat all cancers using a cancer vaccine consisting of only one type of tumor antigen. Diversity of the type or the tissue of cancer cells gives diversity of the type or the amount of a tumor antigen being expressed in the cancer cells. Actually, it has been reported that immunotherapy, using a plurality of peptides (multi-peptide based immunotherapy), is effective in cancer therapy (Non-Patent Reference 9, 10, and 11).

In addition, given that the type of tumor antigen peptide that functions in each individual differs due to the polymorphism of the HLA gene, it is important to identify a tumor antigen peptide that induces and/or activates an antigen-specific cytotoxic T-lymphocyte restricted for each HLA type in order to obtain a high effectiveness in cancer therapy.

Obviously, a cancer vaccine therapy that activates cytotoxic T-lymphocytes using a single tumor antigen will bring some therapeutic effects for a cancer having this tumor antigen. However, in cancer therapy, in order to induce and/or activate antigen-specific cytotoxic T-lymphocytes, and also to obtain high therapeutic effectiveness corresponding to the diversity of cancers, it is important to discover and use numerous novel tumor antigens that correspond to the HLA-restriction and diversity of cancers.

The references cited in the description of this background art are listed below.
Non-Patent Reference 1: *Archives of Surgery*, 1990, Volume 126, pp. 200-205.
Non-Patent Reference 2: *Science,* 1991, Volume 254, pp. 1643-1647.
Non-Patent Reference 3: *Journal of Experimental Medicine,* 1996, Volume 183, pp. 1185-1192.
Non-Patent Reference 4: *Joumal of Immunology,* 1999, Volume 163, pp. 4994-5004.
Non-Patent Reference 5: *Proceedings of the National Academy of Sciences of the United States of America*, 1995, Volume 92, pp. 432-436.
Non-Patent Reference 6: *Science,* 1995, Volume 269, pp. 1281-1284.
Non-Patent Reference 7: *Journal of Experimental Medicine,* 1997, Volume 186, pp. 785-793.
Non-Patent Reference 8: *Nature Medicine,* 1998, Volume 4, pp. 321-327.
Non-Patent Reference 9: *Clinical Cancer Research*, 2001, Volume 7, pp. 3950-3962.
Non-Patent Reference 10: *Joumal of Clinical Oncology,* 2001, Volume 19, pp. 3836-3847.
Non-Patent Reference 11: *Nature Medicine,* 1998, Volume 4, pp. 328-332.

### SUMMARY OF THE INVENTION

One aspect of the present invention is a peptide having the amino acid sequence set forth in any one of SEQ ID NO: 1 to 13 in the Sequence Listing.

One additional aspect of the present invention is a polypeptide having the amino acid sequence set forth in any one of SEQ ID NO: 14 to 18 in the Sequence Listing.

One further aspect of the present invention is a peptide having the amino acid sequence set forth in any one of SEQ ID NO: 1 to 13 in the Sequence Listing, wherein the peptide is recognized by a cytotoxic T-lymphocyte and/or induces a cytotoxic T-lymphocyte.

A still further aspect of the present invention is the aforementioned peptide, wherein being recognized by a cytotoxic T-lymphocyte and/or inducing a cytotoxic T-lymphocyte is being recognized by a cytotoxic T-lymphocyte in an HLA-A2-restricted manner and/or inducing a cytotoxic T-lymphocyte in an H LA-A2-restricted manner.

One additional and further aspect of the present invention is a polypeptide having the amino acid sequence set forth in any one of SEQ ID NO: 14 to 18 in the Sequence Listing, wherein the polypeptide is recognized by a cytotoxic T-lymphocyte and/or induces a cytotoxic T-lymphocyte.

One aspect of the present invention, in addition, is the aforementioned polypeptide, wherein being recognized by a cytotoxic T-lymphocyte and/or inducing a cytotoxic T-lymphocyte is being recognized by a cytotoxic T-lymphocyte in an HLA-A2-restricted manner and/or inducing a cytotoxic T-lymphocyte in an HLA-A2-restricted manner.

One aspect of the present invention is, furthermore, a medicament comprising one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

One still further aspect of the present invention is a cancer vaccine that contains one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

One aspect of the present invention is, furthermore, the aforementioned cancer vaccine, which is used in the treatment of a brain tumor.

In addition, one aspect of the present invention is an agent for inducing a cytotoxic T-lymphocyte that contains one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

Furthermore, one aspect of the present invention is a method for inducing a cytotoxic T-lymphocyte comprising using the use of one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

Furthermore, one aspect of the present invention is a method for inducing a cytotoxic T-lymphocyte comprising the steps of:
i) incubating an antigen-presenting cell that retains HLA-A2 with a peptide having the amino acid sequence set forth in any one of SEQ ID NO: 1 to 13 in the Sequence Listing;
   or
ii) expressing a polypeptide having the amino acid sequence set forth in any one of SEQ ID NO: 14 to 18 in the Sequence Listing in an antigen-presenting cell that retains HLA-A2;
   and
iii) using the cell obtained in the aforementioned step i) or the aforementioned step ii) for stimulating a group of cells that contain a precursor cell of the cytotoxic T-lymphocyte.

Furthermore, one aspect of the present invention is the aforementioned medicament, which is used in the prevention and/or the treatment of multiple sclerosis.

One aspect of the present invention is, in addition, a polynucleotide having a nucleotide sequence that codes for a peptide having the amino acid sequence set forth in any one of SEQ ID NO: 1 to 13 in the Sequence Listing, or a polypeptide having the amino acid sequence set forth in any one of SEQ ID NO: 14 to 18 in the Sequence Listing, or a complementary nucleotide sequence thereof.

One aspect of the present invention is, furthermore, a polynucleotide having a nucleotide sequence set forth in any of SEQ ID NO: 19 to 23 in the Sequence Listing, or a complementary nucleotide sequence thereof.

One still further aspect of the present invention is a polynucleotide having the nucleotide sequence set forth in any one of SEQ ID NO: 19 to 23 in the Sequence Listing, wherein the nucleotide sequence is such that the polypeptide coded by said nucleotide sequence is recognized by a cytotoxic T-lymphocyte, and/or induces a cytotoxic T-lymphocyte, or a complementary nucleotide sequence thereof.

One aspect of the present invention is, in addition, the aforementioned polynucleotide wherein being recognized by a cytotoxic T-lymphocyte and/or inducing a cytotoxic T-lymphocyte is being recognized by a cytotoxic T-lymphocyte in an HLA-A2-restricted manner and/or inducing a cytotoxic T cell in an HLA-A2-restricted manner.

In addition, one aspect of the present invention is a polynucleotide that hybridizes under stringent conditions with any of the aforementioned polynucleotides.

Furthermore, one aspect of the present invention is a recombinant vector that comprises any of the aforementioned polynucleotides.

Moreover, one aspect of the present invention is the aforementioned recombinant vector, wherein the recombinant vector is a recombinant expression vector.

In addition, one aspect of the present invention is a transformant that has been transformed by any of the aforementioned recombinant vectors.

One aspect of the present invention is, in addition, a method for preparing any of the aforementioned peptides or any of the aforementioned polypeptides, wherein the method comprises a step of culturing a transformant that has been transformed by a recombinant expression vector containing any of the aforementioned polynucleotides.

One aspect of the present invention is, furthermore, an antibody that immunologically recognizes any of the aforementioned peptides and/or any of the aforementioned polypeptides.

Moreover, one aspect of the present invention is a method for identifying: a compound that interacts with the aforementioned peptide or the aforementioned polypeptide and/or an HLA-A2 molecule, and at least enhances the recognition of the peptide or the polypeptide by an HLA-A2-restricted cytotoxic T-lymphocyte; and/or a compound that interacts with any of the aforementioned polynucleotides and enhances the expression thereof, wherein the method comprises using at least one of: the aforementioned peptides; the aforementioned polypeptides; any of the aforementioned polynucleotides; any of the aforementioned recombinant vectors; the aforementioned transformants; or the aforementioned antibodies.

In addition, one aspect of the present invention is a compound that is identified by the method described above.

One aspect of the present invention is, in addition, a compound that enhances the recognition of at least one of the aforementioned peptides or the aforementioned polypeptides by an HLA-A2-restricted cytotoxic T-lymphocyte.

One aspect of the present invention is, furthermore, a compound that interacts with any of the aforementioned polynucleotides and enhances the expression thereof.

One still further aspect of the present invention is a pharmaceutical composition for use in cancer therapy that contains an effective dose of at least one of: any of the aforementioned peptides; any of the aforementioned polypeptides; any of the aforementioned polynucleotides; any of the aforementioned recombinant vectors; the aforementioned transformants; the aforementioned antibodies; or any of the aforementioned compounds.

One aspect of the present invention is, furthermore, a pharmaceutical composition for use in the prevention and/or treatment of multiple sclerosis that contains an effective dose of at least one of: any of the aforementioned peptides; any of the aforementioned polypeptides; any of the aforementioned polynucleotides; any of the aforementioned recombinant vectors; the aforementioned transformants; the aforementioned antibodies; or any of the aforementioned compounds.

In addition, one aspect of the present invention is a method for quantitatively and/or qualitatively measuring: any of the aforementioned peptides; any of the aforementioned polypeptides; or any of the aforementioned polynucleotides.

Furthermore, one aspect of the present invention is the use of the aforementioned measurement method, which is used in cancer screening.

A still further aspect of the present invention is a reagent kit comprising at least one of: any of the aforementioned peptides; any of the aforementioned polypeptides; any of the aforementioned polynucleotides; or the aforementioned antibodies.

In addition, one aspect of the present invention is a reagent kit for use in any of the aforementioned methods, wherein the reagent kit comprises at least one of the following: any of the aforementioned peptides; any of the aforementioned polypeptides; any of the aforementioned polynucleotides; or the aforementioned antibodies.

### BRIEF DESCRIPTION OF THE DRAWINGS

In the figures, * indicates that a significant difference (P<0.05) was observed in the two-tailed Student t-test.

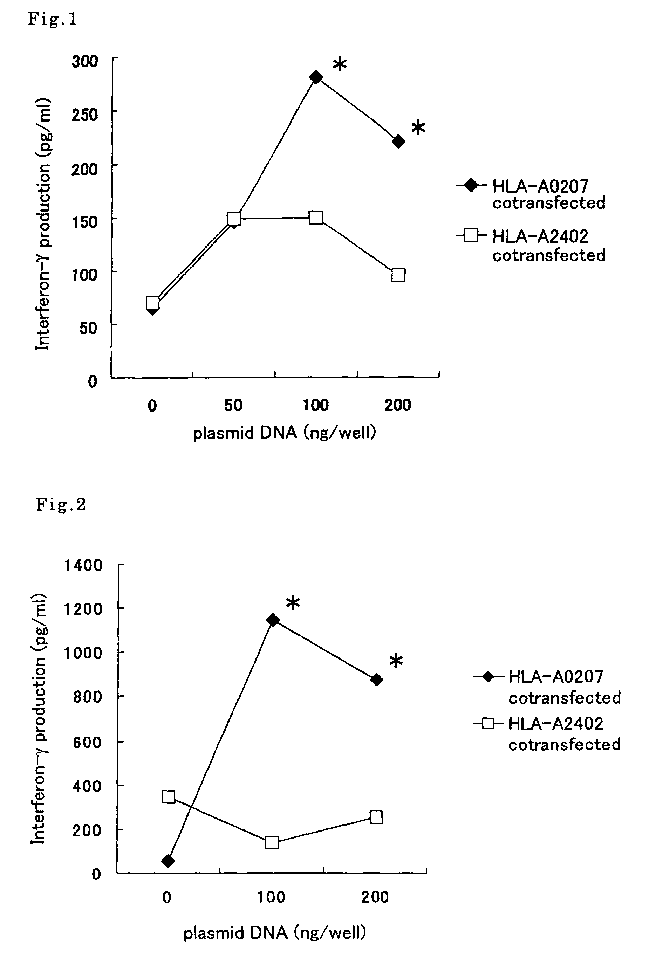

Fig. 1 shows that the cDNA clone 8B6, derived from human glioma cell KNS60, was recognized in a plasmid dose-dependent manner and also in an HLA-A2-restricted manner by a cytotoxic T-lymphocyte strain, OK-CTL, and that it enhanced the production of interferon-γ from the OK-CTL.

Fig. 2 shows that the cDNA clone 2G2, derived from the human glioma cell KNS60, was recognized in a plasmid dose-dependent manner and also in an HLA-A2-restricted manner by the cytotoxic T-lymphocyte strain OK-CTL, and that it enhanced the production of interferon-γ from the OK-CTL.

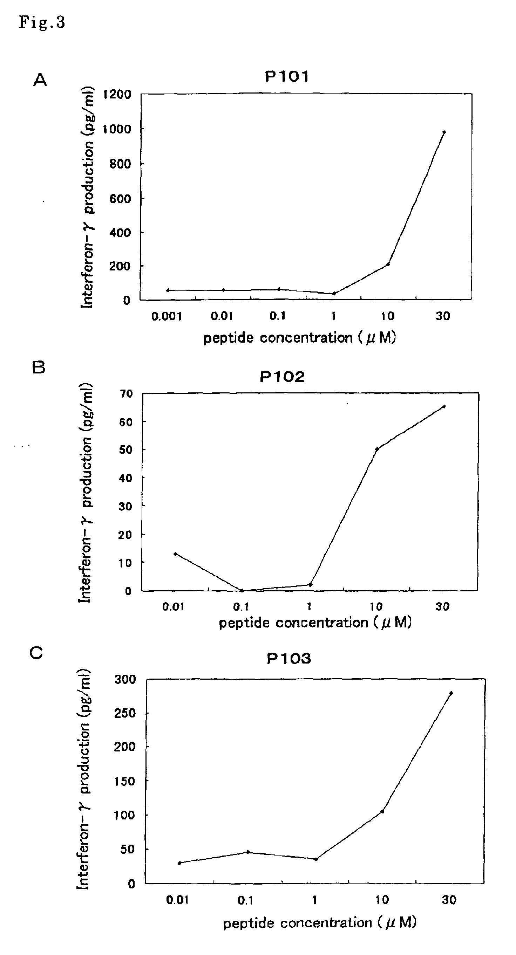

Figs. 3A, 3B, and 3C show, respectively, that P101 (SEQ ID NO: 1), P102 (SEQ ID NO: 2), and P103 (SEQ ID NO: 3) which are peptides derived from clone 8B6, were recognized by the HLA-A2-restricted cytotoxic T-lymphocyte strain OK-CTL in a peptide dose-dependent manner, and that they enhanced the production of interferon-γ from the OK-CTL.

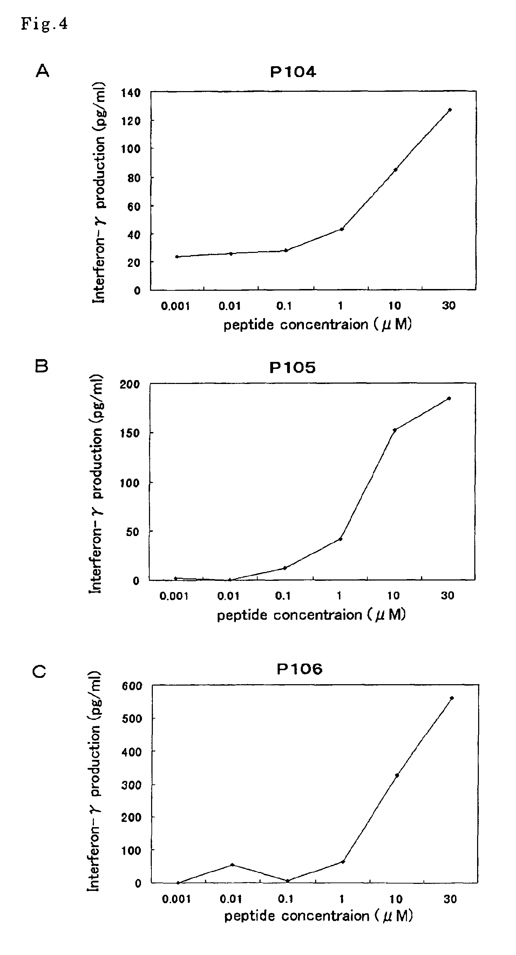

Figs. 4A, 4B, and 4C show, respectively, that P104 (SEQ ID NO: 4 in the Sequence Listing), P105 (SEQ ID NO: 5 in the Sequence Listing), and P106 (SEQ ID NO: 6 in the Sequence Listing) which are peptides derived from clone 2G2, were recognized by the HLA-A2-restricted cytotoxic T-lymphocyte strain OK-CTL in a peptide dose-dependent manner, and that they enhanced the production of interferon-γ from the OK-CTL.

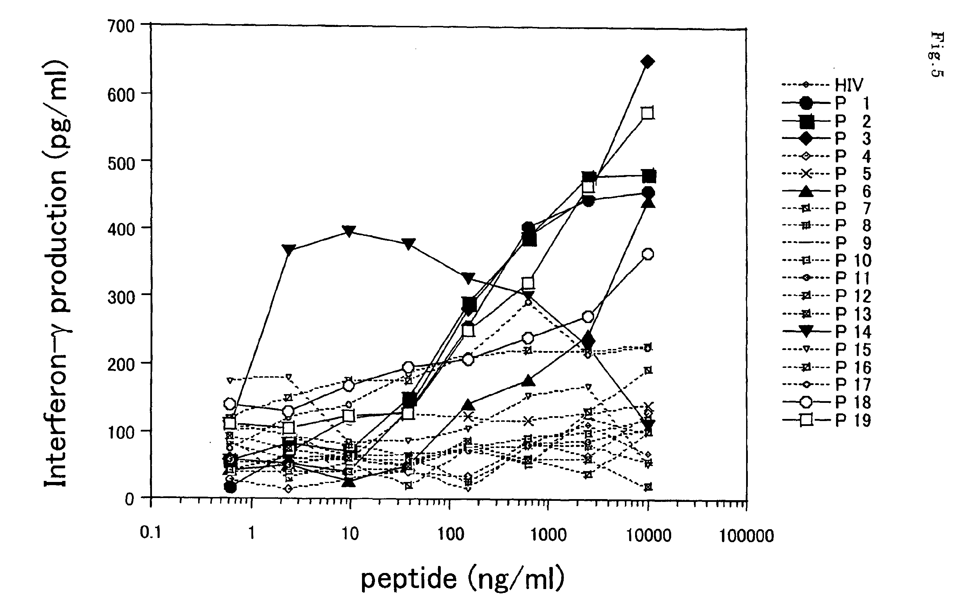

Fig. 5 shows that each of seven peptides (P1, P2, P3, P6, P14, P18, and P19), derived from clone 4G3, clone 7H9, or clone 1B10, were recognized by the HLA-A2-restricted cytotoxic T-lymphocyte strain OK-CTL in a peptide dose-dependent manner, and that they enhanced the production of interferon-γ from the OK-CTL.

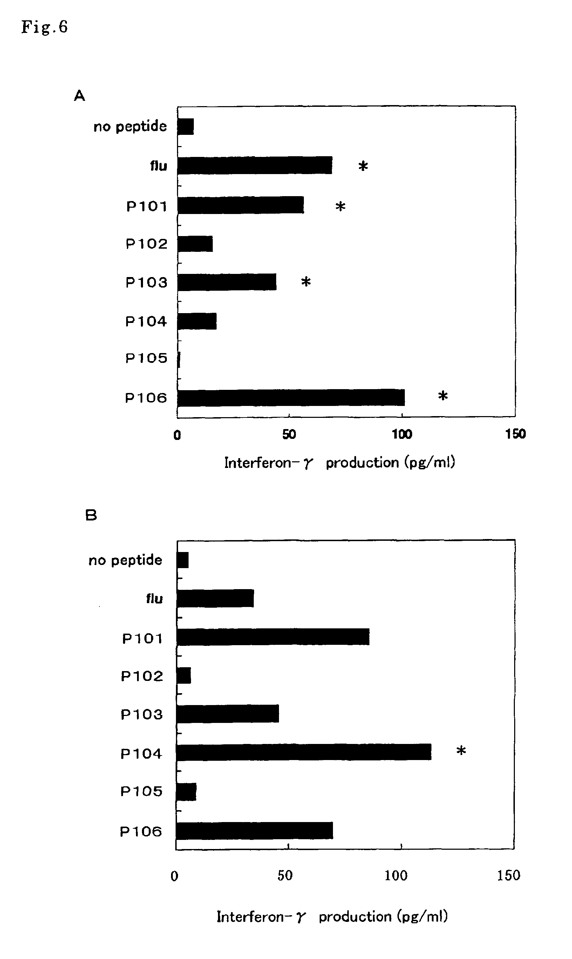

Figs. 6A and 6B show that, when peripheral blood mononuclear cells (PBMC) derived from a metastatic brain tumor patient and PBMC derived from a meningioma patient, respectively, were stimulated with P101 (SEQ ID NO: 1), P102 (SEQ ID NO: 2), and P103 (SEQ ID NO: 3), which are derived from clone 8B6, or P104 (SEQ ID NO: 4 in the Sequence Listing), P105 (SEQ ID NO: 5 in the Sequence Listing), and P106 (SEQ ID NO: 6 in the Sequence Listing), which are peptides derived from clone 2G2, they recognized T2 cells (HLA-A2⁺) that had been pulsed with the each corresponding peptide, and as a result, production of interferon-γ from PBMC was enhanced.

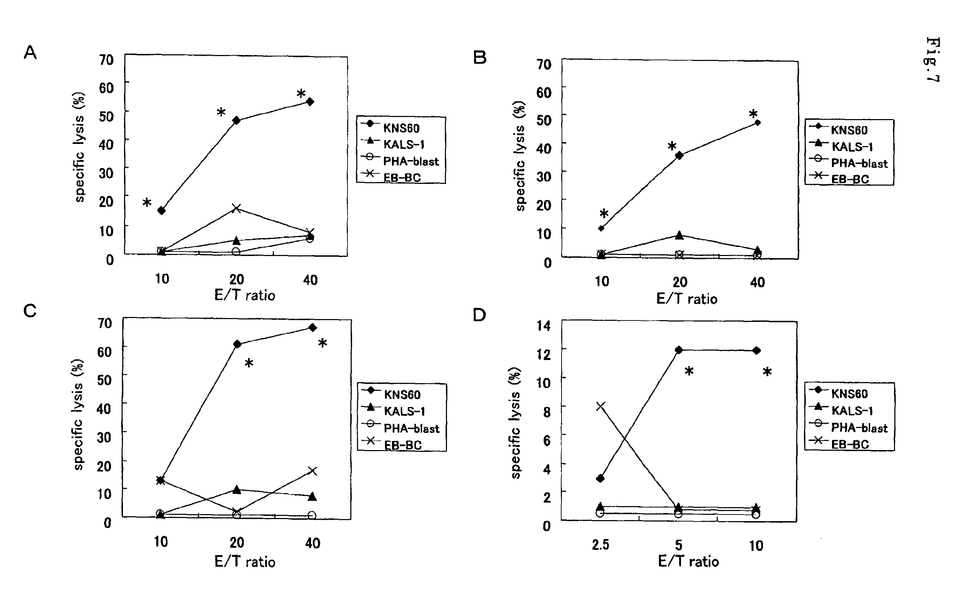

Figs. 7A, 7B, 7C, and 7D show that peripheral blood mononuclear cells, derived from a metastatic brain tumor patient, which were stimulated with peptide P101 (SEQ ID NO: 1 in the Sequence Listing), which is derived from clone 8B6, peptide P103 (SEQ ID NO: 3 in the Sequence Listing), which is derived from clone 8B6, peptide P106 (SEQ ID NO: 6 in the Sequence Listing), which is derived from clone 2G2, and peptide P104 (SEQ ID NO: 4 in the Sequence Listing), which is derived from clone 2G2, respectively recognized HLA-A2⁺ tumor cell strain KNS60, indicated cytotoxic activity, and lysed the tumor cell strain, but they did not recognize HLA-A2⁻ tumor cell strain KALS-1 or any normal cells (PHA-blast and EB-BC).

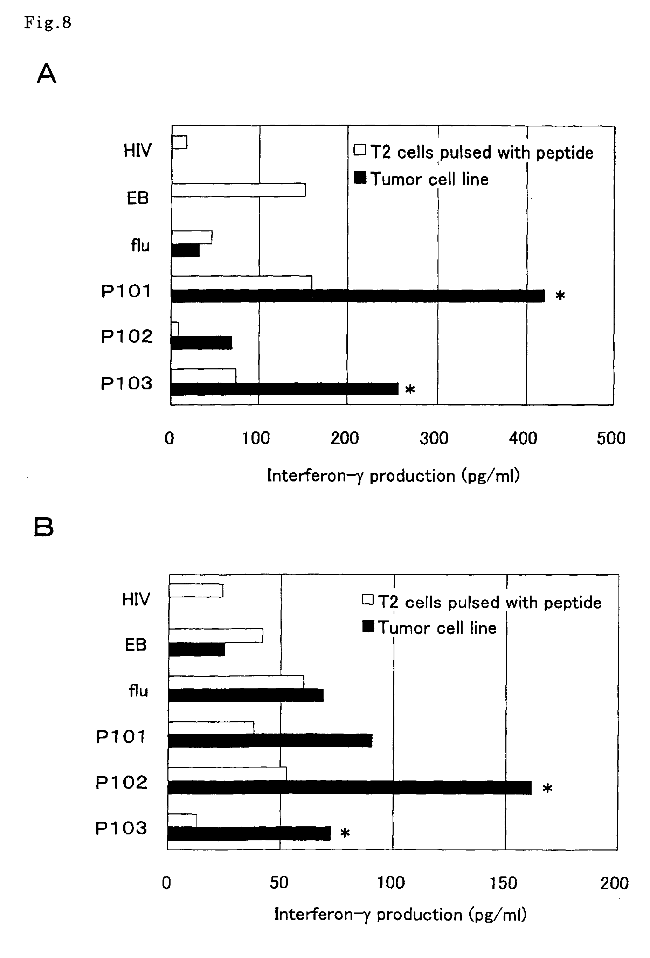

Figs. 8A and 8B show that peripheral blood mononuclear cells (PBMC), derived from different brain tumor patients, which were stimulated with P101 (SEQ ID NO: 1), P102 (SEQ ID NO: 2), or P103 (SEQ ID NO: 3), which are peptides derived from clone 8B6, recognized T2 cells (HLA-A2⁺) that had been pulsed with each corresponding peptide and/or tumor cell strain (HLA-A2⁺), and as a result, production of interferon-γ from the PBMC was enhanced. In these figures, HIV, EB, and flu indicate control peptides derived from an immunodeficiency virus, an Epstein-Barr virus, and an influenza virus, respectively.

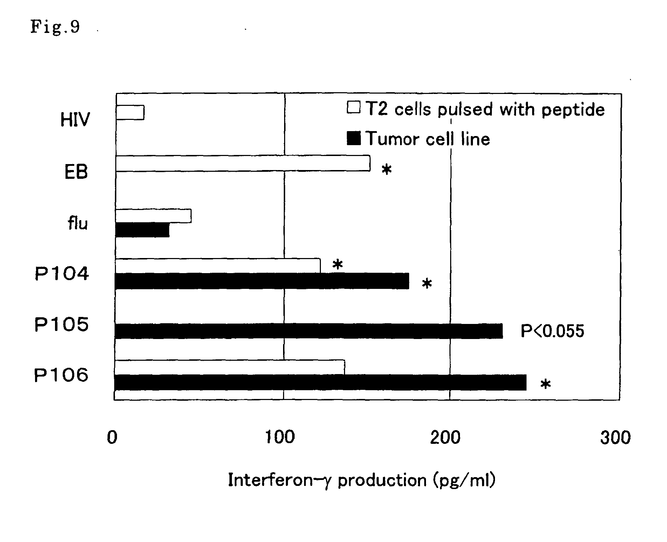

Fig. 9 shows that peripheral blood mononuclear cells (PBMC), derived from a brain tumor patient, when stimulated with P104 (SEQ ID NO: 4 in the Sequence Listing), P105 (SEQ ID NO: 5 in the Sequence Listing), and P106 (SEQ ID NO: 6 in the Sequence Listing), which are peptides derived from clone 2G2, recognized T2 cells (HLA-A2⁺) that had been pulsed with each corresponding peptide and/or tumor cell strain (HLA-A2⁺), and as a result, production of interferon-γ from the PBMC was enhanced. In this figure, HIV, EB, and flu indicate control peptides derived from an immunodeficiency virus, an Epstein-Barr virus, and an influenza virus, respectively.

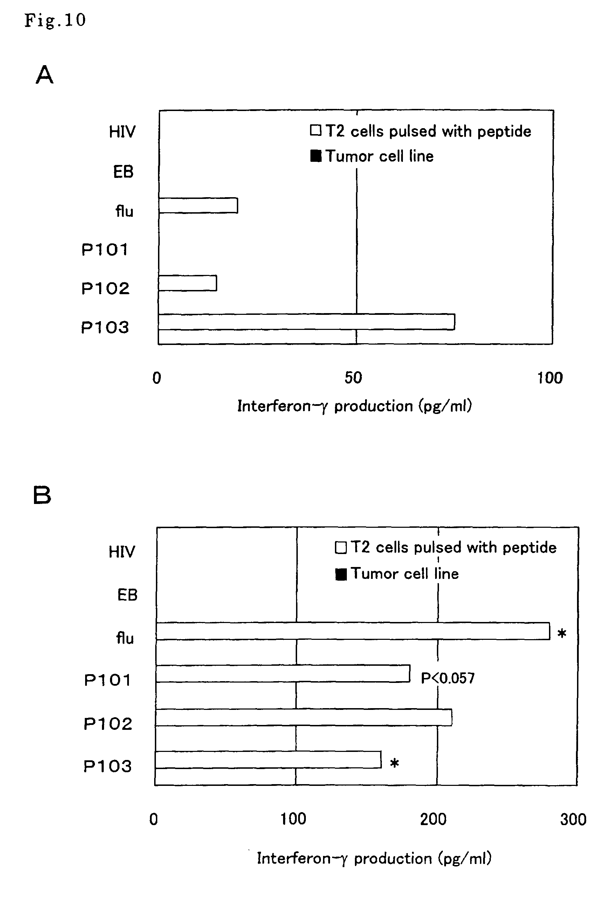

Figs. 10A and 10B show that peripheral blood mononuclear cells (PBMC), derived from different multiple sclerosis patients, when stimulated with P101 (SEQ ID NO: 1), P102 (SEQ ID NO: 2), or P103 (SEQ ID NO: 3), which are peptides derived from clone 8B6, recognized T2 cells that had been pulsed with each corresponding peptide, and as a result, production of interferon-γ from the PBMC was enhanced, but the PBMC did not recognize the tumor cells. In these figures, HIV, EB, and flu indicate control peptides derived from an immunodeficiency virus, an Epstein-Barr virus, and an influenza virus, respectively.

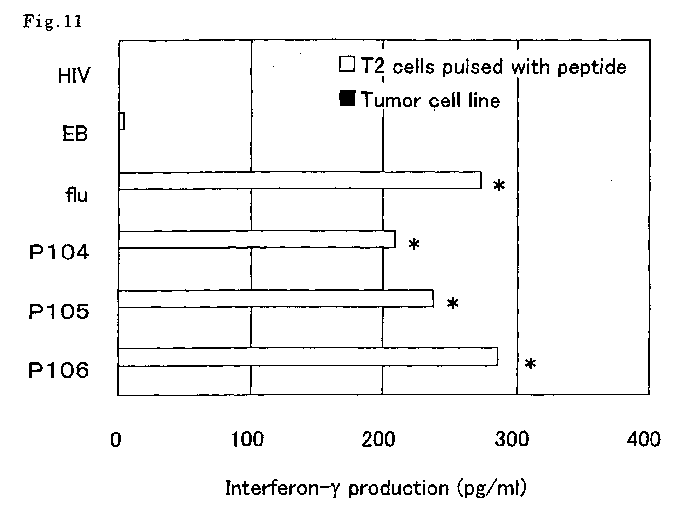

Fig. 11 shows that peripheral blood mononuclear cells (PBMC), derived from a multiple sclerosis patient, when stimulated with P104 (SEQ ID NO: 4 in the Sequence Listing), P105 (SEQ ID NO: 5 in the Sequence Listing), and P106 (SEQ ID NO: 6 in the Sequence Listing), which are peptides derived from clone 2G2, recognized T2 cells that had been pulsed with each corresponding peptide, and as a result, production of interferon-γ from the PBMC was enhanced, but the PBMC did not recognize the tumor cells. In this figure, HIV, EB, and flu indicate control peptides derived from an immunodeficiency virus, an Epstein-Barr virus, and an influenza virus, respectively.

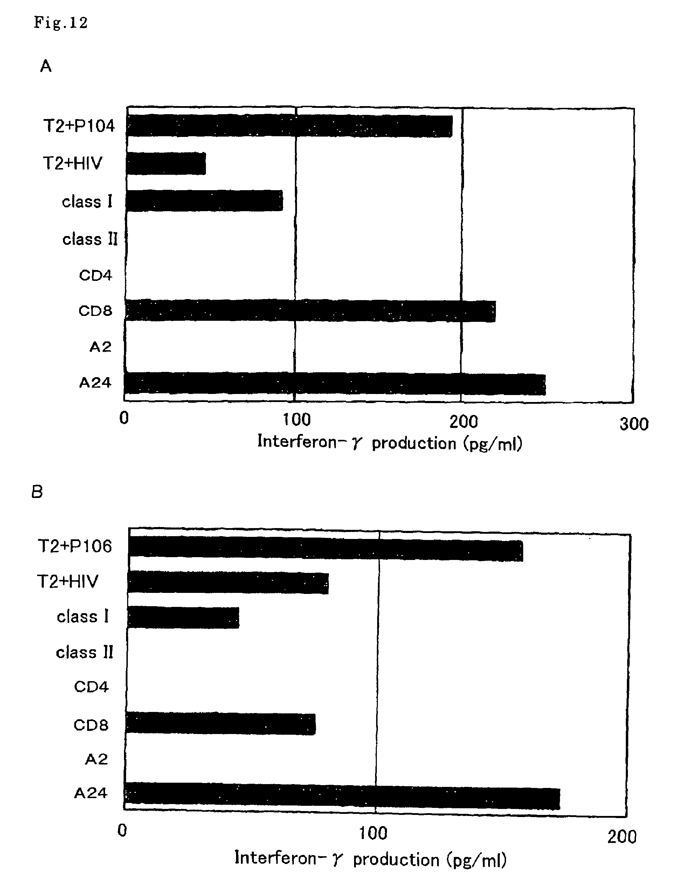

Figs. 12A and 12B show that production of interferon-γ from peripheral blood mononuclear cells, derived from a healthy subject, was inhibited by anti-HLA-class II antibody, anti-CD4 antibody, or anti-HLA-A2 antibody; the peripheral blood mononuclear cells having been stimulated with peptide P104 (SEQ ID NO: 4 in the Sequence Listing) and peptide P106 (SEQ ID NO: 6 in the Sequence Listing), respectively, which are peptides derived from clone 2G2; and the production of interferon-γ having been enhanced by the peripheral blood mononuclear cells recognizing T2 cells that have been pulsed with the corresponding peptide.

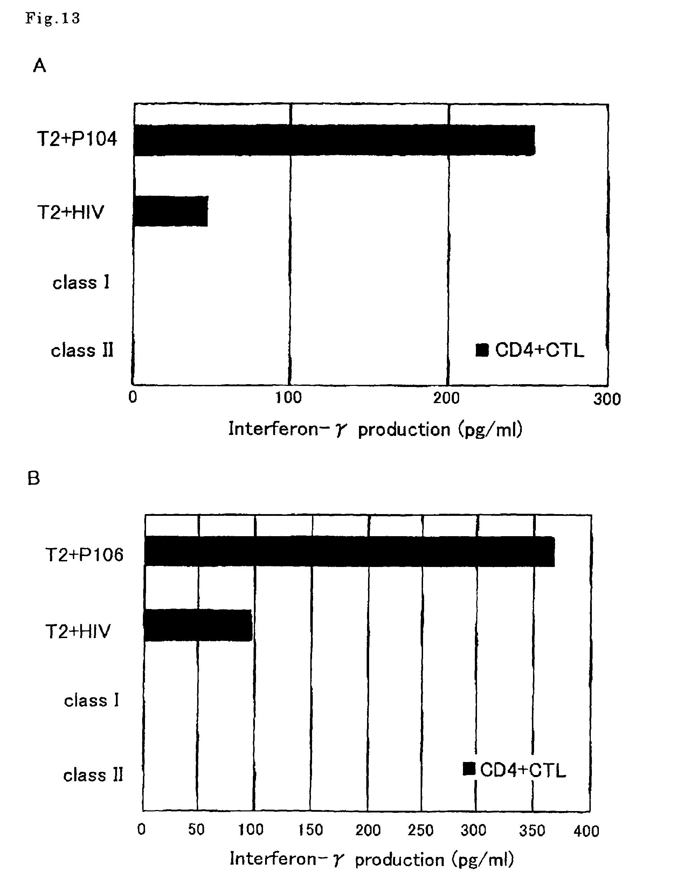

Figs. 13A and 13B show that production of interferon-γ from CD4-positive cells, purified from peripheral blood mononuclear cells derived from a healthy subject, was inhibited by an anti-HLA-class I antibody or an anti-HLA-class II antibody; the peripheral blood mononuclear cells having been stimulated with peptide P104 (SEQ ID NO: 4 in the Sequence Listing) and peptide P106 (SEQ ID NO: 6 in the Sequence Listing), respectively, which are peptides derived from clone 2G2; and the production of interferon-γ having been enhanced by CD4-positive cells recognizing T2 cells that had been pulsed with each corresponding peptide respectively.

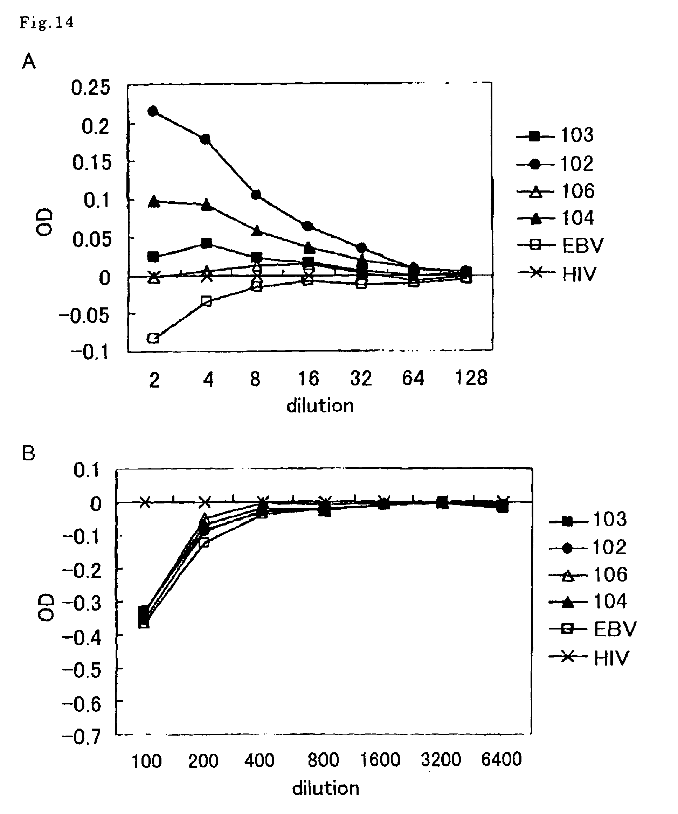

Fig. 14A and 14B show, respectively, the results obtained by quantifying specific IgE antibodies and specific IgG antibodies against P102 (SEQ ID NO: 2) or P103 (SEQ ID NO: 3), which are peptides derived from clone 8B6, or P104 (SEQ ID NO: 4) or P106 (SEQ ID NO: 6), which are peptides derived from clone 2G2, in the serum of a multiple sclerosis patient.

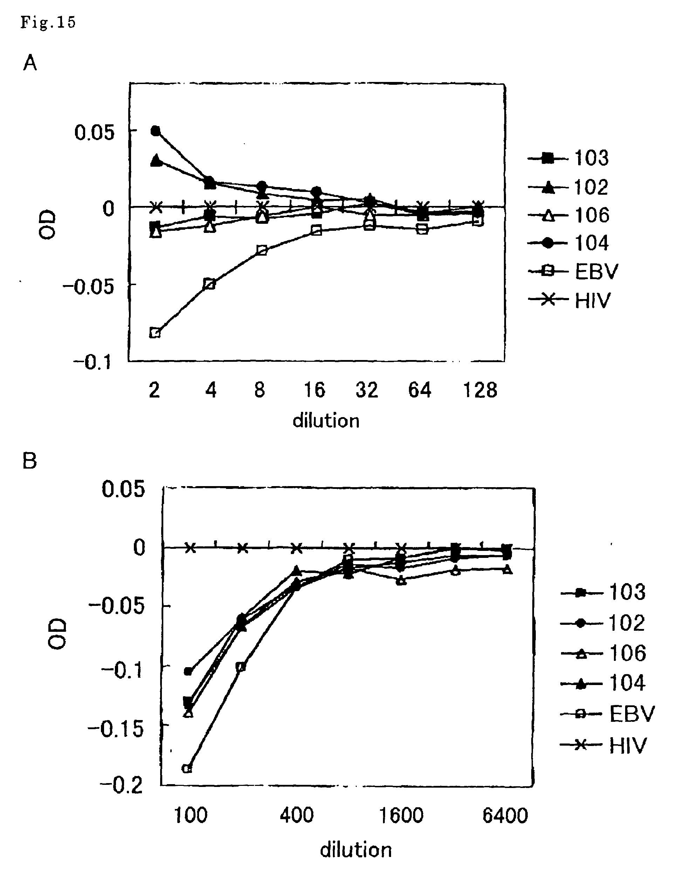

Figs. 15A and 15B show, respectively, the results obtained by quantifying specific IgE antibodies and specific IgG antibodies against P102 (SEQ ID NO: 2) or P103 (SEQ ID NO: 3), which are peptides derived from clone 8B6, or P104 (SEQ ID NO: 4) or P106 (SEQ ID NO: 6), which are peptides derived from clone 2G2, in the serum of a brain tumor patient.

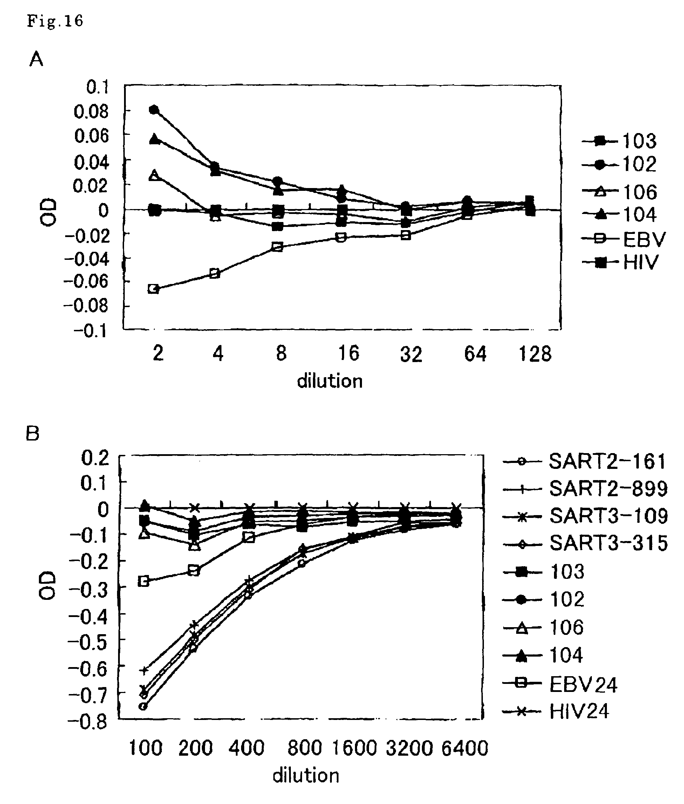

Figs. 16A and 16 show, respectively, the results obtained by quantifying specific IgE antibodies and specific IgG antibodies against P102 (SEQ ID NO: 2) or P103 (SEQ ID NO: 3), which are peptides derived from clone 8B6, or P104 (SEQ ID NO: 4) or P106 (SEQ ID NO: 6), which are peptides derived from clone 2G2, in the serum of a healthy subject.

### DETAILED DESCRIPTION OF THE INVENTION

The present invention claims priority from Japanese Patent Application No. 2001-333219 which is incorporated herein by reference.

In order to understand the present invention, the terminology used in the present specification will first be explained. A tumor antigen means a protein or peptide that a tumor cell possesses, which may be recognized by a tumor-specific cytotoxic T-lymphocyte and/or may induce a cytotoxic T-lymphocyte. In addition, a tumor antigen peptide means a peptide generated by degrading of the tumor antigen inside a tumor cell, and this peptide may be recognized by a tumor-specific cytotoxic T-lymphocyte and/or induce a cytotoxic T-lymphocyte by binding to an HLA molecule and being presented on the surface of the cell. Furthermore, the site of an amino acid sequence that a tumor antigen possesses, which may induce and/or activate a tumor-specific cytotoxic T-lymphocyte, is called a tumor antigen epitope (tumor antigen determinant).

Here, to "recognize" means that a recognizing entity discerns a to-be-recognized target from others and may bind to the recognized target. In particular, in the present specification, recognition of a tumor cell or a tumor antigen peptide by a cytotoxic T-lymphocyte means that a cytotoxic T-lymphocyte binds to a tumor antigen peptide presented by an HLA molecule via a T-cell antigen receptor (hereinafter also abbreviated as TCR). To "activate" means to further enhance or bring into action an entity or a state that has a given activity or effect. In particular, in the present specification, activation of a cytotoxic T-lymphocyte means that a cytotoxic T-lymphocyte generates, for example, IFN-γ as a result of recognizing an antigen presented by an HLA molecule, or that a cytotoxic T-lymphocyte demonstrates a cytotoxic activity against a target cell (also called target) that has been recognized. To "induce" means to generate a given activity or effect from an entity or a state that substantially lacks the activity or the effect. In particular, in the present specification, to induce an antigen-specific cytotoxic T-lymphocyte means to cause a cytotoxic T-lymphocyte that specifically recognizes a given antigen to differentiate and/or to proliferate *in vitro* or *in vivo.* In addition, in the present specification, a "cytotoxic T-lymphocyte inducing agent" means an agent that demonstrates an effect, wherein a state where CD8 positive T-lymphocytes that specifically recognize a given antigen is not present or is present only at an extremely low ratio, is changed to a state where cytotoxic T-lymphocytes that recognize the antigen are present at an extremely high ratio.

In the present specification, a long chain peptide among any of peptides containing two or more amino acids bound to one another through a peptide bond or a modified peptide bond, is called a polypeptide. For instance, in the present specification, proteins are also included as polypeptides. In addition, short chain peptides, also referred to as oligopeptides and oligomers, are simply called peptides. In the following, when an amino acid sequence is represented, it may be represented by one letter, or it may be represented by three letters.

Other technical and scientific terms used herein have the meanings commonly understood by one of ordinary skill in the art to which the present invention pertains, unless otherwise defined. Reference is made herein to various methodologies known to those of ordinary skill in the art. Publications and other materials setting forth such known methodologies to which reference is made are incorporated herein by reference in their entireties.

Hereinafter, the present invention will be described in more detail for the various embodiments of the present invention.

The following detailed description is illustrative, and its purpose is merely explanatory and does not limit the present invention in any way.

The peptide provided in the present invention is a partial peptide that is contained in a polypeptide coded by a cDNA that has been isolated and identified from a cDNA library of KNS60 cells (*Human Cell*, 1990, Volume 3, pp. 255-256), which is a human brain tumor cell strain, using the gene expression cloning method. When the cDNA is introduced into a cell and expressed, the cell is recognized by a tumor-specific cytotoxic T-lymphocyte, and can induce and/or activate the cytotoxic T-lymphocyte. More concretely, when expressed in a cell that possesses HLA-A2, it is recognized by the tumor-specific cytotoxic T-lymphocyte in an HLA-A2-restricted manner, and can induce and/or activate the cytotoxic T-lymphocyte.

The aforementioned peptide that is contained in the polypeptide coded by such cDNA has a characteristic that it is recognized by a tumor-specific cytotoxic T-lymphocyte and can induce and/or activate the cytotoxic T-lymphocyte. More concretely, it is recognized by the tumor-specific cytotoxic T-lymphocyte in an HLA-A2-restricted manner, and can induce and/or activate the cytotoxic T-lymphocyte.

Isolation and identification of the above-mentioned cDNA were carried out as follows. First, an HLA-A2-restricted and tumor-specific cytotoxic T-lymphocyte, which can be activated by recognizing the HLA-A2 and a tumor antigen peptide, was established from tumor-infiltrating lymphocytes (hereinafter abbreviated as TIL) of a colon cancer patient (HLA-A0207/3101) by methods set forth in existing reports (Non-Patent Reference 4). Hereinafter, this cell is referred to as OK-CTL. The cell surface markers of OK-CTL are CD3⁺ CD4- CD8⁺, and antigen recognition thereof is HLA-A gene locus restricted. That is to say, it recognizes an HLA-A2-positive (hereinafter noted as HLA-A2⁺) cell, but does not recognize an HLA-A2-negative (hereinafter noted as HLA-A2⁻) cell. HLA-A2-positive means that the antigen specificity of the HLA-A allele is A2. HLA-A2-negative means that the antigen specificity of the HLA-A allele is other than A2.

Next, COS-7 cells were cotransfected with the cDNA derived from KNS60 tumor cells and HLA-A0207 cDNA, and among the cells wherein the transgenes were expressed, those that enhance the production of interferon-γ (hereinafter may be abbreviated as IFN-γ) from OK-CTL were selected. As a result, five types of cDNA clones (from SEQ ID NO: 19 to 23) that code for gene products that are recognized by the OK-CTL in an HLA-A2-restrictive manner were obtained.

When a homology search was carried out for these nucleotide sequences against existing databases, such as GenBank, human genes that are highly homologous to clone 8B6 (SEQ ID NO: 19) and clone 2G2 (SEQ ID NO: 20) were found; however, regarding three types of genes in total, such as clone 4G3 (SEQ ID NO: 21), clone 7H9 (SEQ ID NO: 22), and clone 1B10 (SEQ ID NO: 23), highly homologous genes were not found (see Table 1). Although the nucleotide sequences of the human genes highly homologous to clone 8B6 (SEQ ID NO: 19) or clone 2G2 (SEQ ID NO: 20) and the deduced amino acid sequences thereof are disclosed, there is no report that they code for tumor antigens, nor are these disclosed in the open database of the National Center for Biotechnology Information (NCBI) when it was searched as of 7 November, 2001.

The nucleotide sequences of clone 8B6 (SEQ ID NO: 19) and clone 2G2 (SEQ ID NO: 20) and the nucleotide sequence of clone 4G3 (SEQ ID NO: 21), clone 7H9 (SEQ ID NO: 22), and clone 1B10 (SEQ ID NO: 23), were registered with the DNA Data Bank of Japan (DDBJ) of the National Institute of Genetics on 24 April, 2001, and on 6 July, 2001, respectively; however, they were not disclosed as of 30 October, 2001.

The genes obtained in the present invention are genes that code for tumor antigens that are recognized by HLA-A2-restricted cytotoxic T-lymphocytes (hereinafter may be abbreviated as CTL). These genes code for the amino acid sequences set forth in SEQ ID NO: 14 to 18, respectively, in the Sequence Listing (see Table 1). These genes, when expressed in cells as mentioned above, are recognized by an HLA-A2-restricted CTL and can activate the CTL.

**Table 1**

| Clone (base pairs: bp) [Accession number] | SEQ ID NO | Polypeptide coded by gene (amino acids) | SEQ ID NO | Active peptide | SEQ ID NO | Amino acid sequence of the peptide | Highly homologous gene [Accession number] |
|---|---|---|---|---|---|---|---|
| 8B6 (2292) [AB060691] | 19 | PP 8B6 (204) | 14 | P 101 | 1 | TIMAFRWVT | 1,3-Nacetylgalactosaminyl transferase (GALT3) [AF154848] |
| | | | | P 102 | 2 | IMSRDLVPRI | |
| | | | | P 103 | 3 | NLLKVNIHI NLLKVNIHI | |
| 2G2 (1392) [AB060692] | 20 | PP 2G2 (201) | 15 | P 104 | 4 | FLPHFQALHV | human ADP-ribosylation factor 4L (ARF4) [NM001661] |
| | | | | P 105 | | ALHVWIGL | |
| | | | | P 106 | | GITFQVWDV | |
| 4G3 (701) [AB065085] | 21 | PP 4G3 (73) | 16 | P 1 | 7 | CLGEEVLET | - |
| | | | | P 2 | 8 | IIIGFFCYT | |
| | | | | P 3 | 9 | GIHLACFVEV | |
| 7H9 (1848) [AB065086] | 22 | PP 7H9 (111) | 17 | P 6 | 10 | ILWKEKNSA | - |
| 1B10 (2039) [AB065087] | 23 | PP 1B10 (111) | 18 | P 14 | 11 | NLVSLFSRYV | - |
| | | | | P 18 | 12 | NQWTEVMFMA | |
| | | | | P 19 | 13 | VMFMATRELL | |

A peptide having the amino acid sequence listed in any one of SEQ ID NO: 1 to 13 in the Sequence Listing was obtained by selecting a peptide that was recognized by the CTL in an HLA-A2-restricted manner from peptides that were designed and synthesized, as peptides conformable to HLA-A2-binding motifs, based on amino acid sequences that are coded by the aforementioned genes or homologous genes thereof. It is known that a tumor antigen peptide that is capable of binding to an HLA-A2 molecule has a motif (regular arrangement) in its amino acid sequence. Thus, an HLA-A2-binding motif was first retrieved from the Internet site <http://bimas.dcrt.nih.gov//molbio/hla_bind/>, and the amino acid sequence that conforms to this motif was specified in the amino acid sequences that are coded by the aforementioned genes, as well as in the amino acid sequence of the gene products from the genes that are highly homologous to the aforementioned genes. Based on this result, different 9- or 10-mer peptides that have the HLA-A2-binding motif were designed and synthesized. T2 cells (HLA-A2⁺), having been pulsed with each of the synthesized peptides, and OK-CTL were co-cultured, and IFN-γ produced from this OK-CTL was measured. Among the synthesized peptides, 13 types of peptides (SEQ ID NO: 1 to 13) were recognized by the OK-CTL in a dose-dependent manner, and they enhanced production of IFN-γ from the OK-CTL. In addition, these peptides induced CTL from the peripheral blood mononuclear cells (hereinafter may be abbreviated as PBMCs) that were obtained from a cancer patient. The induced CTL recognized the target cells, that is, T2 cells (HLA-A2⁺) that had been pulsed with the corresponding peptide, and/or HLA-A2⁺ tumor cells produced IFN-γ, and lysed the target cells. Thus, 13 types of tumor antigen peptides that can induce and/or activate CTL were obtained in the present invention.

Furthermore, among these thirteen types of peptides, when each of the peptides listed as SEQ ID NO: 1 to 6 in the Sequence Listing were co-cultured with the PBMCs obtained from a multiple sclerosis (hereinafter may be abbreviated as MS) patient or a healthy subject, the PBMCs recognized T2 cells (HLA-A2⁺) which were pulsed with the corresponding peptide, and enhanced the production of IFN-γ, but did not recognize HLA-A2⁺ tumor cells. Since recognition of T2 cells, which were pulsed with the peptide, by the stimulated PBMCs was inhibited by anti-CD4 antibodies, and CD4⁺ cells that were purified from the stimulated PBMCs using anti-CD4 antibodies recognized each corresponding peptide, the cells that may recognize each peptide induced by the stimulation of the aforementioned peptide in the peripheral blood mononuclear cells from a healthy subject or a multiple sclerosis patient are thought to be, not CD8⁺ CTL, but CD4⁺ T-lymphocytes. Generally, CD4⁺ T-lymphocytes are known to recognize complexes of MHC class II molecules and peptides on antigen-presenting cells and produce various kinds of cytokines, for instance, IFN-γ. However, recently, a case in which a CD4⁺ cell recognizes an antigen in a class I-restricted manner has been reported (*Cancer Research*, 1999, Volume 59, pp. 6230-6238), and thus there is a possibility that the induced CD4⁺ cells described above recognize antigens in a class I-restricted manner.

Multiple sclerosis is a representative demyelination disease of the central nervous system; with the pathology and the course thereof being clinically and pathologically complex, and demonstrating diversity (Martin, R. *et al*., *Nature Immunology*, 2001, Volume 2, pp. 785-788). In addition, the onset mechanism thereof is complex and has not yet been elucidated; however, it is said to be an autoimmune disease in which encephalitogenic T-lymphocytes (CD4⁺ cells that belong to T-helper 1) that recognize various proteins and peptides in brain cells as antigens participate (Matsumoto, Y. *et al*., *Clinical Immunology,* 1997, Volume 29, pp. 1207-1212; Yamamura, Ryu, *Brain and Nerves,* 2001, Volume 53, pp. 707-713). The antigens recognized by these encephalitogenic T-lymphocytes are diverse, and it is suggested that, for instance, myelin basic protein (MBP), proteolipid protein (PLP), myelin-associated glycoprotein (MAG), glial fibrillary acidic protein (GFAP), and S-100β, as well as myelin oligodendrocyte glycoprotein (MOG), whose encephalitogenicity is apparent, could be the antigens.

Since the cDNAs, that code for the peptides and polypeptides of the present invention from which the peptides were derived, were originated from brain tumor cells, and since PBMC derived from an MS patient that were stimulated by these peptides recognized each corresponding peptide and enhanced the production of IFN-γ, these peptides are thought to participate in MS. In addition, since the present invention revealed that immunoglobulins E (IgE) that recognize these peptides are present in the blood of MS patients, it is possible that stimulation of mast cells by the IgE that recognizes these peptides is related to the pathologic formation of MS.

Several treatments for multiple sclerosis are currently being performed. For example, treatment by administration of interferon-β, which is aimed at suppressing the recurrence and progression via the immunomodulation mechanism, and steroid pulse therapy, which is aimed at treating through anti-inflammatory action and induction of lymphoid cell death are used. Furthermore, in order to suppress the recurrence and progression of MS, a method, wherein immunological tolerance against antigens that are recognized by the T-lymphocytes is induced by peptides that are recognized by encephalitogenic T-lymphocytes or analog peptides thereof, in which a portion of the peptide is substituted with other amino acids, is examined in MS and experimental autoimmune encephalomyelitis (EAE) which is an animal model of MS (Yamamura, *T., Clinical Immunology*, 1997, Volume 29, pp. 1213-1218; Gaur, A., *Science,* 1992, Volume 258, pp. 1491-1494). Immunological tolerance means a state where immunoreactivity against a specific antigen is absent. That is to say, if the immunological tolerance induced in MS, which is one of the autoimmune diseases triggered as a result of the enhancement of the immune response against an autoantigen, it is expected that MS can be suppressed from progressing, ameliorated, and prevented from recurring. The peptides of the present invention can be used to induce such immunological tolerance. That is to say, it is possible that the peptides of the present invention can be used in the prevention and/or treatment of MS.

Accordingly, the present invention provides polypeptides that are coded by the aforementioned genes obtained from KNS60 tumor cells. Preferably, these are one or more polypeptides having any one of the amino acid sequences set forth in SEQ ID NO: 14 to 18 in the Sequence Listing. Since these polypeptides are recognized by an HLA-A2-restricted antigen-specific CTL, they can be used as tumor antigens that induce and/or activate the CTL. Furthermore, these polypeptides can be used as materials to obtain tumor antigen peptides by specifying the tumor antigen epitopes.

The peptides of the present invention can be obtained, for instance, by designing peptides conformable to the HLA-A2-binding motif based on the amino acid sequences of the aforementioned polypeptides, and then selecting from the designed peptides that are recognized by CTL in an HLA-A2-restricted manner. The peptides may be those that bind to HLA-A2 and are presented on the surface of the antigen-presenting cells, and the peptides have the characteristics of a tumor antigen epitope that is recognized by CTL, and is a peptide having 5 or more, preferably 7 or more, more preferably 9 or 10, amino acid residues. Even more preferable is a peptide having any one of the amino acid sequences set forth in SEQ ID NO: 1 to 13 in the Sequence Listing. Since these peptides are recognized by antigen-specific CTL in an HLA-A2-restricted manner, they can be used to induce and/or activate HLA-A2-restricted antigen-specific CTL.

The aforementioned polypeptides or peptides may be used singly or used in combination of two or more in order to induce and/or activate CTL. Since CTL are a plurality of cell groups that recognize various antigens, it is recommended that preferably two or more of these be used in combination.

In addition, polypeptides or peptides, which have one or more amino acids with mutation(s), such as deletion, substitution, addition, or insertion, are also included in the scope of the present invention. Preferable are polypeptides or peptides having such mutation(s) and which are recognized by CTL, for instance, at least by HLA-A2-restricted CTL. Peptides having such mutation(s) may be those that exist naturally, or those in which one or more mutation(s) has been introduced. The means for introducing mutation(s), such as deletion, substitution, addition, or insertion, are known, and the method by Ulmer (*Science,* 1983, Volume 219, p. 666 et seq.) can, for instance, be used. Seeing that fundamental characteristics (physical properties, activity, or immunological activity and the like) of these peptides are not changed by the introduction of such mutation(s), reciprocal substitution among, for example, homologous amino acids (polar amino acids, non-polar amino acids, hydrophobic amino acids, hydrophilic amino acids, positively charged amino acids, negatively charged amino acids, aromatic amino acids, and the like) is readily inferred. Furthermore, these usable peptides can be altered to the extent that no significant functional alteration is involved, such as modifying their constituent amino group or carboxyl group and the like.

The polynucleotides of the present invention are polynucleotides having the nucleotide sequences that respectively code for the peptides having any one of the amino acid sequences set forth in SEQ ID NO: 1 to 13 or the polypeptides having any one of the amino acid sequences set forth in SEQ ID NO: 14 to 18 in the Sequence Listing, or complementary nucleotide sequences thereof. More preferable are polynucleotides having any one of the nucleotide sequences set forth in SEQ ID NO: 19 to 23 in the Sequence Listing, or complementary nucleotide sequences thereof. Furthermore, the polynucleotides may have at least 15 or more, preferably 21 to 30 or more, nucleotide sequences corresponding to the regions that code for tumor antigen epitopes among the amino acid sequences of the polypeptides pertaining to the present invention, or complementary nucleotide sequences thereof. In the present invention, polynucleotides having complementary nucleotide sequences may be designated as complementary strands. Selection of such a useful polynucleotide and determination of the nucleotide sequence thereof can be carried out, for example, by employing well-known protein expression systems to confirm the ability of the expressed peptide or polypeptide to induce and/or activate CTL.

Furthermore, polynucleotides that hybridize to the above-mentioned polynucleotides under stringent conditions are also included in the scope of the present invention. If a DNA molecule is taken as a representative example of a polynucleotide molecule, "a DNA molecule that hybridizes to a DNA molecule under stringent conditions" can be obtained by methods described in, for instance, Sambrook *et al*., eds., *Molecular Cloning: A Laboratory Manual,* 1989, Cold-Spring Harbor Laboratory Press, Cold-Spring Harbor, New York. Here, "hybridizing under stringent conditions" means that, under conditions where, for instance, after heating in a solution of 6×SSC (final concentration of 150 mM NaCl and 15 mM trisodium citrate), 0.5% SDS, and 50% formamide at 42°C, and washing in a solution of 0.1 × SSC and 0.5% SDS at 68°C, a positive hybridization signal is still observed.

When expressed in cells that possess HLA-A2, the aforementioned polynucleotides can be recognized by HLA-A2-restricted and antigen-specific CTL and/or can induce the CTL. In addition, the polynucleotides have a poly (A) structure at the 3' end thereof; however, the number of poly (A) does not influence the site encoding the amino acids which act as tumor antigens, and therefore, there is no particular restriction on the number of poly (A) that the polynucleotides possess.

The aforementioned polynucleotides all provide genetic information useful in preparing the polypeptides or peptides of the present invention, or may be used as nucleic acid reagents or standards.

Recombinant vectors are obtained by integrating the aforementioned polynucleotides into adequate vector DNAs. Vector DNAs to be used are selected appropriately depending on the host type and purpose of use. Vector DNAs may be those obtained by extracting vector DNAs naturally present, and also may be those that lack a part of the DNAs other than that required for multiplication. For instance, chromosome-, episome-, and virus-derived vectors; for instance bacterial plasmid-derived, bacteriophage-derived, transposon-derived, yeast episome-derived, insertion element-derived, yeast chromosomal element-derived; virus-derived vectors, such as from baculovirus, papovavirus, SV40, vaccinia virus, adenovirus, fowlpox virus, pseudorabies virus, and retrovirus, as well as vectors combining these; vectors derived from genetic elements of plasmid and bacteriophage, such as, cosmid and phagemid and the like, may be exemplified. In addition, expression vectors, cloning vectors, and the like may be used depending on the purpose.

A recombinant vector has, as components, the target gene sequence and gene sequences that carry information regarding replication and regulation, such as a promoter, ribosome binding site, terminator, signal sequence, and enhancer. The vector may be created by combining the components by using methods well known in the art. As the method for integrating the polynucleotides of the present invention into the previously mentioned vector DNAs, methods well known to one skilled in the art may also be adopted. For instance, a method may be used in which suitable restriction endonucleases are selected; DNA is cleaved at specific sites using these and then mixed with DNA to be used as a vector, which has been treated in the same way, followed by re-ligating with a ligase. Otherwise, a desired recombinant vector may be obtained by ligating an adequate linker to the target polynucleotide followed by inserting the resultant into a multi-cloning site of a vector suitable for a purpose.

Transformants can be obtained by introducing the vector DNA, into which the aforementioned polynucleotide has been integrated, into a host that is well-known to one skilled in the art by methods that are well-known to one skilled in the art. *Escherichia coli,* yeast, *Bacillus subtilis,* insect cells, or animal cells may be cited as examples of hosts. In preferred systems, when carrying out transfection, the method of integration into chromosomes may be cited if gene stability is to be considered; however, for simplicity, auto-replicating systems that use extranuclear genes may be employed. Introduction of a vector DNA into a host cell can be carried out by standard methods, for instance, as described in Sambrook *et al*. eds., *Molecular Cloning: A Laboratory Manual,* Second Edition, Cold-Spring Harbor Laboratory Press, 1989. Concretely, calcium phosphate transfection, DEAE-dextran-mediated transfection, microinjection, cationic lipid-mediated transfection, electroporation, transduction, scrape loading, ballistic introduction, infection, and the like can be cited.

The polypeptides or peptides of the present invention can be provided if an expression vector is used as a vector DNA to be introduced into the aforementioned transformant. The transformant having an expression vector DNA, into which the aforementioned polynucleotide has been integrated, can be cultured under culture conditions that are optimal for each host and well-known to one skilled in the art. The culture may be performed using as an indicator the action of the polypeptides or peptides of the present invention expressed by the transformant, for example, the action of at least inducing and/or activating CTL; or the quantity of polypeptides or peptides generated inside or outside the host; or a passage culture or a batch culture may be performed using the quantity of transformant in the culture medium.

The polypeptides or peptides of the present invention may be manufactured by genetic engineering techniques as mentioned above, using the aforementioned vectors or transformants. In addition, they may also be manufactured by any method known in general peptide chemistry. For instance, methods described in *Peptide Synthesis,* 1975, Maruzen Co., Ltd., or *Peptide Synthesis,* 1996, Interscience, New York can be used. Naturally, many known methods can be used.

In terms of purification and collection of the polypeptides or peptides of the present invention, it is possible to purify and collect them by using gel filtration chromatography, ion column chromatography, or affinity chromatography and the like, or combinations thereof, or by a fractionation means based on a difference in solubility using ammonium sulfate or alcohol and the like, using their characteristics and biological activity as indicators, for instance whether or not the peptides are at least recognized by CTL and/or induce CTL. More preferably a method can be used, in which polypeptides or peptides are specifically adsorbed and collected by using polyclonal antibodies or monoclonal antibodies, which can be prepared against the polypeptides or the peptides based on the information of their amino acid sequences.

The antibodies can be created using the aforementioned polypeptides or peptides as antigens. The antigen can be the polypeptide or peptide, or fragments thereof, and can be constituted by at least eight, preferably at least ten, more preferably at least twelve, even more preferably fifteen or more amino acids. In order to create antibodies that are specific to the polypeptides and/or peptides, it is preferable to use regions having amino acid sequences that are unique to the polypeptides or peptides. This amino acid sequence does not necessarily need to be homologous to the amino acid sequences of the polypeptides or peptides. Sites that are exposed to the exterior of the tertiary structure of the polypeptides or peptides are preferred. Even if the amino acid sequences of the exposed sites are discontinuous in the primary structure, it suffices that they be amino acid sequences that are continuous on the exposed site. There are no particular limits on the antibodies, as long as they immunologically bind to or recognize the polypeptides and/or peptides. Whether or not this binding or recognition occurs is determined by the well-known antigen-antibody-binding reaction.

Any antibody preparation method well known in the art can be used to produce the antibodies. For instance, they can be obtained by administering to an animal a polypeptide or peptide of the present invention with or without linking such to a carrier, in the presence or absence of an adjuvant, to induce immunity, such as a humoral response and/or cell-mediated response. The carrier is not limited in particular, as long as it does not exert a harmful effect by itself on the host and is capable of enhancing antigenicity; for example, cellulose, polymeric amino acids, albumin, key hole limpet hemocyanin (KLH), and the like. Examples of the adjuvant can be a Freund complete adjuvant (FcA), Freund incomplete adjuvant (FIA), Ribi (MPL), Ribi (TDM), Ribi (MPL+TDM), *Bordetella pertussis* vaccine, muramyl dipeptide (MDP), aluminum adjuvant (ALUM), and combinations thereof. For animals to be immunized, mouse, rat, rabbit, goat, horse, and the like may be suitably used.

The polyclonal antibody is obtained from the serum of the animals subjected to the aforementioned immunization means, by any suitable method for collecting antibodies. As a preferable means, the immuno-affinity chromatography method can be cited.

The monoclonal antibody can be produced by collecting antibody-producing cells (for example, a lymphocyte derived from a spleen or a lymph node) from the animals subjected to the aforementioned immunization, followed by introducing a well-known transformation technique with a permanently proliferating cell (for example, myeloma strain such as P3X63Ag8 cells.) For example, an antibody-producing cell can be fused with a permanently proliferating cell by methods that are well-known to one skilled in the art to create a hybridoma, which can then be cloned, followed by selecting hybridoma producing an antibody that recognizes specifically the aforementioned polypeptides and/or peptides. The antibody can then be collected from a culture solution of the hybridoma.

The polyclonal antibody or monoclonal antibody thus obtained, which can recognize and bind to the aforementioned polypeptides and/or peptides, can be used as a purification antibody, reagent, or labeling marker for the polypeptides or the peptides.

The aforementioned polypeptides or peptides, polynucleotides coding therefor, complementary strands thereof, cells that have been transformed based on the information of the amino acid sequence and nucleotide sequence thereof, or antibodies that immunologically recognize the above, provide, independently or in combination, an effective means of identifying a substance that may enhance recognition of the polypeptide or peptide by CTL. The identification method of the present invention can be established using pharmaceutical screening systems that are well-known in the art. For instance, a substance that enhances recognition of the polypeptides or peptides of the present invention by CTL can be screened by stimulating CTL with antigen-presenting cells which have been pulsed with a tumor antigen peptide, or antigen-presenting cells in which a tumor antigen is expressed, followed by establishing an experimental system to measure recognition of the tumor antigen peptide or the tumor antigen by the CTL and/or activation of the CTL, and examining the test substance. Antigen-presenting cells can include cells that retain HLA-A2, for example, cell strains that retain HLA-A2, or more specifically, T2 cells and the like. Or, even cells that do not possess HLA-A2 can be used by genetically introducing HLA-A2 cDNA so that an HLA-A2 molecule is expressed on the cell surface. Pulsing the tumor antigen to antigen-presenting cells can be performed by co-culturing antigen-presenting cells and tumor antigen with any suitable method. The tumor antigen can be expressed in antigen-presenting cells by introducing the polynucleotide that codes for the antigen into the cell by any suitable technique. Examples of CTL include HLA-A2-restricted CTL strain or HLA-A2-restricted CTL line, such as OK-CTL and the like. Recognition of the tumor antigen peptide or the tumor antigen by the CTL and/or activation of the CTL can be easily determined by measuring IFN-γ production from the CTL. This experimental system is to describe one identification method, and the identification method of the present invention is not restricted thereby.

The present invention also includes the compound obtained by the aforementioned identification. The compound may be a compound that interacts with a polypeptide or a peptide of the present invention, for example, a peptide having the amino acid sequence of any one of those of SEQ ID NO:1 to SEQ ID NO:13 in the Sequence Listing, or a polypeptide having the amino acid sequence of any one of those of SEQ ID NO:14 to SEQ ID NO:18 in the Sequence Listing, and/or an HLA-A2 to enhance recognition of the polypeptide or the peptide by HLA-A2-restricted CTL. In addition, a compound or the like that interacts with a polynucleotide of the present invention and enhances expression thereof is also within the scope of the present invention. A compound screened in such a manner can be prepared as a pharmaceutical composition by selecting it while taking into account the balance between biological usefulness and toxicity.

The polypeptides or peptides provided in the present invention can be used as tumor antigens or tumor antigen peptides, in order to induce and/or activate CTL that are antigen-specific in an HLA-A2-restricted manner. That is to say, medicaments can be used that contain one or more polypeptides selected from the aforementioned polypeptides and/or one or more peptides selected from the aforementioned peptides. Methods for inducing CTL which are characterized by the use of one or more polypeptides/peptides selected from the aforementioned polypeptides/peptides, as well as agents for inducing CTL which can contain one or more polypeptides/peptides selected from the aforementioned polypeptides/peptides, are also included within the scope of the present invention. The aforementioned method for inducing CTL can include, as one embodiment thereof, a step of pulsing a peptide of the present invention to an antigen-presenting cell, or a step of having a polypeptide of the present invention expressed in the antigen-presenting cells. A further step may include stimulating a cell group that contains CTL precursor cells, using the antigen-presenting cells obtained by any one of the aforementioned steps. Examples of antigen-presenting cells include cells that retain HLA-A2, such as cell strains that retain HLA-A2, more concretely, T2 cells and the like. Furthermore, cells that ordinary do not possess HLA-A2 can be used by genetically introducing HLA-A2 cDNA to make them express an HLA-A2 molecule on the cell surface. Pulsing the peptide to antigen-presenting cells can be performed by co-culturing antigen-presenting cells and tumor antigen by any suitable method. The polypeptide can be expressed in the cells by introducing the polynucleotide that codes therefor into an antigen-presenting cell by way of a general genetic engineering technique. Cell groups that contain CTL precursor cells are, for instance, peripheral blood cells, or more preferably, peripheral blood mononuclear cells.

In addition, a pharmaceutical composition can be provided which contains an effective dose of at least one of the following: the polypeptides or the peptides of the present invention; polynucleotides coding for the polypeptides and complementary strands thereof; recombinant vectors created based on the information of the amino acid sequences and nucleotide sequences thereof; cells that are transformed by the recombinant vectors; antibodies that immunologically recognize the polypeptides/peptides; compounds that interact with the polypeptides or the peptides, and/or, HLA-A2, and enhance recognition of the polypeptides or peptides by CTL; or compounds that interact with the polynucleotides and enhance expression thereof, when used alone or in combination of a plurality thereof. For instance, the pharmaceutical composition could be useful in the treatment of cancers, such as the treatment of brain tumors. Considering that the HLA-A2 allele is found in approximately 40% of Japanese, approximately 53% of Chinese, approximately 49% of North American Caucasians, approximately 38% of South American Caucasians, and approximately 23% of African Blacks, the pharmaceutical composition of the present invention can be effective on a multitude of patients.

Concretely, medicaments containing, for instance, one or more polypeptides/peptides selected from the aforementioned polypeptides/peptides and pharmaceutical compositions that contain effective doses of one or more polypeptides selected from the aforementioned polypeptides and/or one or more peptides selected from the aforementioned peptides, can be used as so-called cancer vaccines. The term cancer vaccine as used herein means a drug that selectively damages tumor cells by inducing and/or enhancing a specific immune response against the tumor cells. The dosage thereof can be determined with appropriate modifications, according to the extent of recognition of the polypeptides or peptides by CTL. For example, in general it is between 0.01 and 100 mg/day/adult human, or preferably 0.1 and 10 mg/day/adult human as an active principle. This can be administered once every few days to every few months. Administration may be carried out according to well-known methods for administrating a peptide for medical use, preferably subcutaneously, intravenously, or intramuscularly. In order to induce and/or enhance the immune response during administration, the polypeptide and/or the peptide of the present invention may be used with or without linking such to a carrier in the presence or absence of an appropriate adjuvant. The carrier is not limited in particular, as long as it exerts no harmful effect by itself on the human body and is capable of enhancing antigenicity; cellulose, polymeric amino acids, albumin, and the like can be given as examples. Adjuvants may be those used in general for peptide vaccine inoculation, and a Freund incomplete adjuvant (FIA), aluminum adjuvant (ALUM), *Bordetella pertussis* vaccine, mineral oil, and the like can be given as examples. In addition, the formulation can be suitably selected by applying a suitable well known method for formulating a peptide.

Otherwise, an effective cancer vaccine effect can be obtained also by, with the aforementioned polypeptide or peptide, inducing and/or activating CTL in the mononuclear cell fraction collected from the peripheral blood of a patient and then returning the fraction of interest into the blood of the patient. Culture conditions, such as mononuclear cell concentration, polypeptide or peptide concentration, culture time, and the like, can be determined by simply repeating experiments. A substance with ability to enhance the growth of lymphocytes, such as interleukin-2, may also be added during culturing.

The aforementioned polypeptides or peptides can be effectively used alone or in combination as a cancer vaccine. Since it has been reported that multi-peptides based immunotherapies are effective (Non-Patent References 9, 10, and 11), and since the CTL of a cancer patient is a group of cells that recognize a plurality of tumor antigens, rather than using one type of polypeptide or one type of peptide as a cancer vaccine, higher effectiveness may be obtained by using a plurality of types in combination for use as a cancer vaccine.

The polynucleotides coding for the polypeptides or peptides of the present invention, or more preferably coding for the peptides, and complementary strands thereof, are useful in gene therapy of cancers; and brain tumors, for instance. There is a method in which vectors containing these polynucleotides can be directly introduced to the body. Furthermore, there is a method in which, after collecting cells from humans, the vectors can be extracorporeally introduced. Retrovirus, adenovirus, vaccinia virus, and the like are known as vectors, however, the retroviral system is recommended. For viruses, nonreplicative ones can be used. The dosage can be determined by adding appropriate modifications according to the extent of recognition of the polypeptides or peptides by the CTL, in general, it can be between 0.1 µg and 100 mg/day/adult human, or preferably between 1 µg and 50 mg/day/adult human as the content of DNAs that code for the polypeptides or peptides of the present invention.

In addition, the polypeptides and the peptides of the present invention were identified from a cDNA library derived from brain tumor cells, and the PBMC which was derived from a multiple sclerosis (MS) patient and stimulated by these peptides, for example, by the peptides having any one of the amino acid sequences listed as SEQ ID NO: 1 to 6 in the Sequence Listing, recognized each corresponding peptide and enhanced IFN-γ production. Furthermore, IgE which recognizes these peptides was detected in the serum of MS patients. These indicate that there is a possibility of these polypeptides and peptides participating in the pathologic formation of MS. Therefore, by using these polypeptides or peptides, preferably peptides, it is possible to prevent and/or treat MS.

For instance, by using the peptides of the present invention and inducing immunological tolerance in MS patients, it is possible to inhibit the progression of, ameliorate, and prevent the recurrence of MS. The dosage, administration period, and administration method of the peptides, as well as the form of administration are adapted for induction of immunological tolerance, and can be determined according to prescriptions generally applied for an antigen peptide therapy against autoimmune diseases. Furthermore, necessary modifications can be performed depending on the degree of seriousness and the like of the particular pathology. Generally, in order to induce immunological tolerance by peptides, it is necessary to administer in large quantities; for instance, between 0.01 and 100 mg/day/human. The administration method should be performed similarly to the well-known administration methods for medically used peptides, and preferably, it is performed via subcutaneous administration, intravascular administration, intranasal administration, or intramuscular administration. During administration, the peptide may be used singly, or may be used together with a suitable well-known adjuvant. For the induction of immunological tolerance, a single peptide may be used, or a plurality of peptides may be used in combination. In addition, analog peptides resulting from the introduction of one to several amino acid substitutions in a peptide, acylated peptides, or peptides bound to MHC class II molecules and the like may be used to increase the effect of immunological tolerance induction.

In addition, the polypeptides of the present invention, for instance, polypeptides having an amino acid sequence listed as SEQ ID NO: 14 or 15 of the Sequence Listing, can also be used in the prevention and/or treatment of MS. Furthermore, it is possible to prevent and/or treat MS by inserting the polynucleotides that code for the peptides or polypeptides of the present invention and complementary strands thereof into a vector, and expressing the peptides or polypeptides in cells or *in vivo.* In addition, the aforementioned pharmaceutical composition can be used in the prevention and/or treatment of MS.

The aforementioned polypeptide or peptide, polynucleotide that codes for the polypeptide or the peptide, and the complementary strand thereof, as well as the antibody that immunologically recognizes the polypeptide or the peptide, can be used singly as a diagnostic marker, reagent, and the like. When used as reagents, they may contain a substance, such as a buffering solution, salt, stabilization agent, and/or antiseptic agent. In addition, the present invention also provides a reagent kit comprising one or more containers that are filled with one or more kinds of these reagents. Furthermore, for formulations, suitable formulation means can be used that are well-known for peptides, polypeptides, polynucleotides, antibodies, and the like.

The abovementioned reagents and reagent kits can be used in the aforementioned screening methods of the present invention. In addition, they can be used for quantitatively and/or qualitatively measuring the polypeptides or peptides of the present invention, or polynucleotides coding for any one thereof. This measuring method can be established using methods that are well-known to those skilled in the art. As examples of methods that can be used, a radio immunoassay, competitive binding assay, Western blot analysis, ELISA, and the like can be given. In addition, in terms of nucleic acids, it is possible to detect and quantify them at the RNA level using, for instance, amplification, PCR, RT-PCR, RNase protection, Northern blotting, and other hybridization methods.

The aforementioned reagents, reagent kits, and measurement methods can be used in the detection method of diseases related to the expression or activation of the polypeptides or peptides of the present invention. As examples of diseases of interest, cancer diseases and the like, more preferably cancer diseases in which the cancer is HLA-A2 positive, even more preferably brain tumors, can be given.

As examples of samples to be measured, cells derived from individuals, for instance, blood, urine, saliva, spinal fluid, tissue biopsy, or necropsy materials and the like, can be given. In addition, the nucleic acids to be measured can be obtained from each of the aforementioned samples by nucleic acid preparation methods that are well-known in the art. For the nucleic acid, genomic DNA can be used directly for detection, or it may be enzymatically amplified prior to analysis by PCR or other amplification methods. RNA or cDNA may be used in a similar manner. In addition, deletions and insertions can be detected by changes in the sizes of the amplification products, in comparison with a normal genotype. In addition, point mutations can be identified by hybridizing the amplified DNA to a labeled DNA that codes for the aforementioned polypeptide.

With a sample derived from an individual, a disease can be detected, for instance, by detecting the presence of the corresponding nucleic acid to the polynucleotide that codes for the polypeptide of interest; by determining the quantity of the nucleic acid; and/or identifying the mutation of the nucleic acid. This nucleic acid can be detected by using its interaction with, and its responsiveness to, the polynucleotide that codes for the polypeptide of interest. Furthermore, a disease can be detected by determining the *in vivo* distribution of the polypeptide or peptide in the individual; by detecting the presence of the polypeptide or peptide; and/ or by determining the quantity of the polypeptide or peptide; or by detecting the mutation of the polypeptide or peptide.

Furthermore, the aforementioned diseases can be examined and diagnosed by qualitatively or quantitatively measuring the polypeptides or peptides of the present invention, or the nucleic acid coding therefor, as diagnostics markers. That is to say, by using the aforementioned detection method, a method for examining or diagnosing the diseases can further be performed.

### EXAMPLES

The present invention will be described more concretely in the following, by way of examples; however, the present invention is not limited to these examples.

### Example 1: Establishment of HLA-A2-restricted CTL

An HLA-A2-restricted tumor-specific cytotoxic T-lymphocyte strain was established from tumor-infiltrating lymphocytes (TIL) of a colon cancer patient (HLA-A0207/3101, HLA-B46/51, HLA-Cw1), according to a method described in the literature (*Intemational Journal of Cancer,* 1999, Volume 81, pp. 459-466; Non-Patent Reference 4). First, TIL obtained from a colon cancer patient were cultured for more than 50 days with the addition of 100 U/ml of recombinant human interleukin-2 (IL-2). Every 7 culture days, a portion of these IL-2-activated TIL were collected, co-cultured with various tumor cells or normal cells, and the CTL activity thereof was assayed by measuring the production of IFN-γ and with a test for Cr⁵¹ released from the tumor cells (Non-Patent Reference 4). Measurement of IFN-γ was performed by enzyme-linked immunosorbent assay (ELISA). At day 58 of culturing, OK-CTL was obtained, which is a subline that demonstrates a tumor-specific cytotoxic activity in an HLA-A2-restricted manner. The phenotype was CD3⁺CD4⁻CD8⁺ for 80% of the OK-CTL, the phenotype of the remaining 20% being CD3⁺CD4⁺CD8⁻.

OK-CTL recognized HLA-A0201⁺ pancreatic adenocarcinoma cell Panc-1, HLA-A0201⁺ colon adenocarcinoma cell SW620, HLA-A0206⁺ esophageal squamous cell carcinoma (SCC) cell KE3, HLA-A0207⁺ oral SCC cell CA9-22, HLA-A2⁺ astrocytoma cell U251, and HLA-A2⁺ glioma cell KNS60, and produced IFN-γ. In addition, it demonstrated sufficient cytotoxic activity. However, it did not demonstrate cytotoxic activity against HLA-A2⁻ tumor cells, autologous Epstein-Barr virus (EBV)-transformed B-cell (hereinafter may be abbreviated as EB-BC), and autologous phytohaemagglutinin (PHA) blastoid T-lymphocyte (Autologous PHA-blastoid T-lymphocytes; hereinafter may be abbreviated as PHA-blast). In addition, OK-CTL lysed all the HLA-A2⁺ tumor cells (HLA-A0201⁺ breast adenocarcinoma cell R27, primary hepatocellular carcinoma cell HAK-2, melanoma cell SK-MEL-5 and astrocytoma cell SF126; HLA-A0206⁺ pulmonary adenocarcinoma cell PC9, as well as pulmonary adenocarcinoma cell 1-87;and HLA-A0207⁺ cervical SCC cell OMC-4) that were examined. Recognition of these cells by OK-CTL and the resulting production of IFN-γ were inhibited by anti-HLA class I monoclonal antibody (mAb), anti-CD8 mAb, or anti-HLA-A2 mAb, but were not inhibited by other mAbs. This shows that OK-CTL recognizes tumor cells in an HLA-A2-restricted manner and demonstrates cytotoxic activity.

### Example 2: Isolation and identification of cDNA clones coding for tumor antigens

The genes coding for tumor antigens that were recognized by OK-CTL obtained in Example 1 were isolated and identified from a cDNA library of glioma cell KNS60 that was derived from human brain tumor cells according to a known method (*Journal of Experimental Medicine,* 1998, Volume 187, pp. 277-288). First, the poly (A)⁺ RNA from KNS60 tumor cells (deposited as IF050357 at the Institute for Fermentation) was prepared according to methods in the art. The poly (A)⁺ RNA obtained was converted into cDNA, ligated to a Sal I adapter, and inserted into the expression vector pCMV-SPORT-2 (Invitrogen). In addition, each of the cDNAs of HLA-A0207, HLA-A2402, and HLA-A2601 were amplified by reverse transcription polymerase chain reaction (hereinafter abbreviated as PCR) and cloned into the eucaryotic cell expression vector pCR3 (Invitrogen).

The cDNA clones obtained from the KNS60 tumor cells were pooled into pools of 100 clones each, and then 100 ng of the cDNA pooled in each well of a U-shaped 96-well plate, and 100 ng of HLA-A0207 cDNA, HLA-A2402 cDNA, or HLA-A2601 cDNA were incubated in 100 µl of a 1:200 mixture of lipofectoamine/Opti-MEM (Invitrogen) for 30 minutes. An amount of 50 µl from this mixture was added to COS-7 cells (1×10⁴) and incubated for 6 hours in a U-shaped 96-well plate for co-transfection. Next, RPMI-1640 culture medium containing 10% FCS was added and cultured for 2 days, and OK-CTL (2×10⁵) were added into each well. After incubating for an additional 18 hours, 100 µl of supernatant was collected, and the IFN-γ produced was measured by ELISA, so as to screen the pools of the cDNA library. At this time COS-7 cells in which no gene was introduced was used as target cells for a negative control, and the production of IFN-γ by OK-CTL was examined. The value of IFN-γ produced was subtracted from each measurement value as the background.

After confirming reproducibility of the pools from the cDNA library of KNS60 tumor cells that enhanced production of IFN-γ from CTL, clones were individually taken up from each cDNA pool whose reproducibility had been verified, and screening was performed by the same method as previously described, so as to select clones derived from independent pools that were recognized by CTL. Furthermore, the dose dependency of the clones obtained was verified by the same method as previously described, and five types of clone were finally obtained. When each of these five types of cDNA clone was co-transfected with the HLA-A0207 cDNA into COS-7 cells, these clones were recognized by OK-CTL and enhanced production of IFN-γ from OK-CTL in a dose-dependent manner. However, when these cDNA clones were co-transfected with HLA-A2402 cDNA or HLA-A2601 cDNA, no enhancement of IFN-γ production by OK-CTL was observed. This makes it clear that these five types of cDNA clone code for tumor antigens that are recognized by OK-CTL in an HLA-A2-restricted manner. The results for clone 8B6 and clone 2G2 are shown in Fig. 1 and Fig. 2, respectively, as representative examples. The same results were obtained for the other clones.

Determination of the nucleotide sequences of the aforementioned five types of cDNA clones, that is, clone 8B6, clone 2G2, clone 4G3, clone 7H9, and clone 1B10, was carried out by the dideoxynucleotide sequencing method using a DNA sequencing kit (Perkin-Elmer) and the ABIPRISM® 377 DNA Sequencer (Perkin-Elmer), (SEQ ID NO: 19 to 23). Furthermore, the amino acid sequence (SEQ ID NO: 14 to 18) encoded by each cDNA clone was deduced from the nucleotide sequence. In addition, a homology search was carried out against the GenBank for the nucleotide sequence of each clone obtained. These results are shown in Table 1 mentioned above.

### Example 3: Peptide preparation and CTL activation test

Tumor antigen peptides were obtained from the genes isolated and identified in Example 2 that code for tumor antigens. First, using a computer, a search for motifs that may bind to the HLA-A2 molecule was carried out for the amino acid sequences coded by each of these genes, or for those coded by genes that are highly homologous to these genes <http://bimas.dcrt.nih.gov//mo)bio/h)a_bind/>. Based on the results, differing 9-mer or 10-mer peptides were designed and synthesized by a method well known in the art. Peptides having a purity of 70% or more were obtained thereby.

Each of the synthesized peptides (0.001 µM to 30 µM or 0.1 ng/ml to 100 µg/ml) was incubated with T2 cells expressing HLA-A2 molecules on the cell surface in a form that does not bind to peptides (*Cancer Research,* 1994, Volume 54, pp. 1071-1076) for 2 hours at 37°C under conditions of 5% CO₂-95% air, to make the peptide of interest bind to the HLA-A2 molecule expressed on the cell surface. The T2 cells, pulsed with each peptide in this way, were used as the target cells (T). In addition, the OK-CTL obtained in Example 1 were used as effector cells (E). Target cells amounting to 1×10⁴ and effector cells amounting to 1×10⁵ were mixed (E/T ratio=10) and incubated for 18 hours. After incubation, 100 µl of supernatant was collected, and IFN-γ was measured by ELISA. The quantity of IFN-γ produced by CTL against T2 cells that were not pulsed with a peptide was taken as the background and subtracted from each measurement value.

As a result, the 13 types of peptide were respectively recognized by OK-CTL in a dose-dependent manner and enhanced production of IFN-γ from OK-CTL. These peptides are P101 (SEQ ID NO: 1), P102 (SEQ ID NO: 2), and P103 (SEQ ID NO: 3) derived from clone 8B6; P104 (SEQ ID NO: 4), P105 (SEQ ID NO: 5), and P106 (SEQ ID NO: 6) derived from clone 2G2; P1 (SEQ ID NO: 7), P2 (SEQ ID NO: 8), and P3 (SEQ ID NO: 9) derived from clone 4G3; P6 (SEQ ID NO: 10) derived from clone 7H9; as well as P14 (SEQ ID NO: 11), P18 (SEQ ID NO: 12), and P19 (SEQ ID NO: 13) derived from clone 1B10. The results for the three types of peptide derived from clone 8B6 and the three types of peptide derived from clone 2G2 are shown in Fig. 3 and Fig. 4, respectively, and the results for the seven types of peptide derived from clone 4G3, clone 7H9, and clone 1B10 are shown in Fig. 5. In Fig. 5, the peptides that were recognized by CTL and/or induced CTL are shown by the solid lines. In addition, the peptide derived from human immunodeficiency virus (hereinafter abbreviated as HIV), which was used as a negative control, is a peptide that may bind to the HLA-A2 molecule. It was not recognized by CTL and did not enhance production of IFN-γ by CTL.

### Example 4: Induction of CTL by peptides from peripheral blood mononuclear cells of cancer patients

Among the 13 types of peptide obtained in Example 3, three peptides, P101 (SEQ ID NO: 1), P102 (SEQ ID NO: 2), and P103 (SEQ ID NO: 3), which were derived from clone 8B6, as well as three peptides, P104 (SEQ ID NO: 4), P105 (SEQ ID NO: 5), and P106 (SEQ ID NO: 6), which were derived from clone 2G2, were examined for their ability to induce HLA-A2-restricted CTL from PBMC obtained from an HLA-A2⁺ brain tumor patient or healthy subject.

PBMC were prepared by methods of the art from the blood of a metastatic brain tumor patient (hereinafter referred to as Patient Case 1), a meningioma patient (hereinafter referred to as Patient Case 2), or 2 healthy subjects. The PBMC amounting to 1×10⁵ were respectively incubated with 10 µM of each peptide in each well of a 96-well U-shaped microculture plate (Nunc) to which 200 µl of a culture medium (consisting of 45% RPMI-1640 culture medium, 45% AIM-V® culture medium (Invitrogen), 100 U/ml of IL-2, 0.1 mM of MEM non-essential amino acid solution (Invitrogen), and 10% fetal calf serum (FCS)) had been added. At 4 days and 7 days of culturing, half the culture medium was removed and replaced with a culture medium of the composition containing the corresponding peptide described above.

At 10 days culture, the cells were collected, washed, and used as effector cells. T2 cells were pulsed with each corresponding peptide, and used as target cells. Both cells were mixed and cultured by the same method as in Example 3, and the quantity of IFN-γ produced was measured. In so doing, the effector cells were paired with the target cells in such a way that the peptide used to induce the effector cells and the peptide pulsed to T2 cells were the same. The results for Patient Case 1 and Patient Case 2 are shown in Fig. 6A and Fig. 6B, respectively. In Patient Case 1, the three peptides, P101 (SEQ ID NO: 1), P103 (SEQ ID NO: 3), and P106 (SEQ ID NO: 6) enhanced production of IFN-γ from PBMC. Furthermore, P104 (SEQ ID NO: 4) in particular enhanced production of IFN-γ in Patient Case 2. These peptides induced HLA-A2-restricted CTL from the PBMC of brain tumor patients. However, neither peptide induced CTL from the PBMC of healthy subjects. Meanwhile, the peptides having the HLA-A2-binding motif, which were derived from an influenza virus (hereinafter may be abbreviated as flu) and used as positive controls, enhanced production of IFN-γ from PBMC in Patient Case 1 with significance. No significance was observed in the enhancement of production of IFN-γ from PBMC in Patient Case 2. Thus, the fact that there are peptides that do not induce CTL from PBMC in brain tumor cancer patients when cultured under the aforementioned conditions, even though the peptides can activate OK-CTL, and the fact that there are cases in which CTL are induced and cases in which CTL are not induced, even though the same peptide is used, may be attributed to CTL precursors present in the peripheral blood of cancer patients. These precursors being a plurality of cell groups capable of recognizing a variety of antigens. Therefore, even if a peptide is not capable of inducing CTL from the PBMC of the brain tumor patient, despite being recognized by OK-CTL and enhancing IFN-γ production thereby, there is still ample possibility of inducing CTL in another patient.

Furthermore, the cells stimulated with the peptide and cultured for 10 days were cultured for a further 10 days, and then the cytotoxic activity against various target cells (HLA-A2⁺) was measured by a standard 6-hour ⁵¹Cr release test. The measurement was carried out by varying the effector cell/target cell (E/T) ratio; the results obtained were expressed as the percentage of specific lysis. KNS60 tumor cells, KALS-1 tumor cells, autologous Epstein-Barr transformed B cells (EB-BC), and PHA-blasts were used as target cells.

The results were that all of the PBMC derived from Patient Case 1 whose IFN-γ production was accelerated by P101 (SEQ ID NO: 1), P103 (SEQ ID NO: 3), or P106 (SEQ ID NO: 6), specifically lysed KNS60 tumor cells (Fig. 7A, Fig. 7B, and Fig. 7C, respectively). In addition, the aforementioned PBMC derived from Patient Case 2 whose IFN-γ production was accelerated by P104 (SEQ ID NO: 4) also specifically lysed KNS60 tumor cells (Fig. 7D). This makes it clear that the aforementioned peptide can induce HLA-A2-restricted tumor-specific cytotoxic T-lymphocytes from the PBMC of cancer patients.

### Example 5: Induction of CTL by peptides from peripheral blood mononuclear cells of cancer patients

Among the 13 types of peptide obtained in Example 3, three peptides, P101 (SEQ ID NO: 1), P102 (SEQ ID NO: 2), and P103 (SEQ ID NO: 3), which were derived from clone 8B6, as well as three peptides, P104 (SEQ ID NO: 4), P105 (SEQ ID NO: 5), and P106 (SEQ ID NO: 6), which were derived from clone 2G2, were examined for their ability to induce CTL in an HLA-A2-restricted manner from PBMC obtained from HLA-A2⁺ brain tumor patients.

The PBMC were prepared by methods of the art from the blood of three primary brain tumor patients and one metastatic brain tumor patient. The PBMC obtained were stimulated by incubation with each peptide in the same way as in Example 4, and were cultured by the same method as in Example 3 using T2 cells that were pulsed with each corresponding peptide or KNS60 tumor cells as target cells, followed by measuring the quantity of IFN-γ produced. When T2 cells that were pulsed with the peptides were used as target cells, the PBMC was paired with the T2 cells in such a way that the peptide used to stimulate PBMC and the peptide pulsed to T2 cells were the same.

Representative results for P101 (SEQ ID NO: 1), P102 (SEQ ID NO: 2), and P103 (SEQ ID NO: 3) are shown in Fig. 8, and representative results for P104 (SEQ ID NO: 4), P105 (SEQ ID NO: 5), and P106 (SEQ ID NO: 6) are shown in Fig. 9. The PBMC that were stimulated by P101 (SEQ ID NO: 1) and P103 (SEQ ID NO: 3) in Patient Case 3 (Fig. 8A), by P102 (SEQ ID NO: 2) and P103 (SEQ ID NO: 3) in Patient Case 4 (Fig. 8B), as well as by P104 (SEQ ID NO: 4) and P106 (SEQ ID NO: 6) in Patient Case 5 (Fig. 9) recognized T2 cells that were pulsed with each corresponding peptide and/or HLA-A2⁺ tumor cells, and enhanced production of IFN-γ. That is to say, these peptides induced HLA-A2-restricted CTL from the PBMC of brain tumor patients.

### Example 6: Peptide recognition in peripheral blood mononuclear cells of multiple sclerosis patients

Among the 13 types of peptide obtained in Example 3, three peptides, P101 (SEQ ID NO: 1), P102 (SEQ ID NO: 2), and P103 (SEQ ID NO: 3), which were derived from clone 8B6, as well as three peptides, P104 (SEQ ID NO: 4), P105 (SEQ ID NO: 5), and P106 (SEQ ID NO: 6), which were derived from clone 2G2, were examined for their ability to induce CTL from PBMC obtained from multiple sclerosis patients having HLA-A2 as the HLA type and healthy subjects, in the same manner as in Example 5.

Representative results for P101 (SEQ ID NO: 1), P102 (SEQ ID NO: 2), and P103 (SEQ ID NO: 3) are shown in Fig. 10, and representative results for P104 (SEQ ID NO: 4), P105 (SEQ ID NO: 5), and P106 (SEQ ID NO: 6) are shown in Fig. 11. The PBMC that were stimulated by P103 (SEQ ID NO: 3) in MS Patient Case 6 (Fig. 10A), by P101 (SEQ ID NO: 1), P102 (SEQ ID NO: 2), and P103 (SEQ ID NO: 3) in MS Patient Case 7 (Fig. 10B), as well as by P104 (SEQ ID NO: 4), P105 (SEQ ID NO: 5), and by P106 (SEQ ID NO: 6) in MS Patient Case 8 (Fig. 11) recognized T2 cells that were pulsed with each corresponding peptide and enhanced production of IFN-γ. However, HLA-A2⁺ tumor cells were not recognized, and production of IFN-γ was not observed. In addition, the stimulated PBMC did not show cytotoxic activity against the aforementioned tumor cells. The same results were obtained for the PBMC obtained from the healthy subjects.

Multiple sclerosis is a representative disease associated with demyelination in the central nervous system. The onset mechanism thereof has not yet been elucidated; however, it is said to be an autoimmune disease in which encephalitogenic T-lymphocytes participate. These T lymphocytes recognize a variety of proteins and peptides that are present in brain cells as antigens, such as, for instance, MBP, PLP, MOG, MAG, or S-100β. Given that the peptides that were used in the present example are coded for by genes that were derived from a brain tumor, and given that PBMC derived from MS patients that were stimulated with these peptides recognized each corresponding peptide and enhanced production of IFN-γ, there is a possibility that these peptides and the polypeptides from which the peptides were derived participate in MS.

### Example 7: Analysis of cells induced from the peripheral blood mononuclear cells of a multiple sclerosis patient by peptides

Using P101 (SEQ ID NO: 1), P102 (SEQ ID NO: 2), P103 (SEQ ID NO: 3), P104 (SEQ ID NO: 4), P105 (SEQ ID NO: 5), and P106 (SEQ ID NO: 6), cells that can recognize these peptides were induced from PBMC that were obtained from the multiple sclerosis patient and the healthy subject in Example 6. Next, an analysis of these cells was attempted.

First, PBMC from the healthy subject were cultured and stimulated with P104 (SEQ ID NO: 4) or P106 (SEQ ID NO: 6) in the same way as in Example 5. The cells that had been stimulated with peptides recognized T2 cells that were pulsed with each corresponding peptide and produced IFN-γ. The results for P104 (SEQ ID NO: 4) are shown in Fig. 12A, and the results for P106 (SEQ ID NO: 6) are shown in Fig. 12B. The recognition was not inhibited by anti-CD8 antibody or anti-A24 antibody; however, it was inhibited weakly by anti-class I antibody, and completely by anti-CD4 antibody, anti-A2 antibody, and anti-class II antibody. In addition, a peptide derived from HIV was used as the negative control for the peptides.

When CD4⁺ cells, which were purified by well-known methods using anti-CD4 antibodies from the cells that had been stimulated by each peptide, were co-cultured with T2 cells that had been pulsed with each corresponding peptide, production of IFN-γ was observed (Fig. 13A: P104 (SEQ ID NO: 4) and Fig. 13B: P106 (SEQ ID NO: 6)). This IFN-γ production was completely inhibited by the anti-class I antibody and by the anti-class II antibody.

It is thus probable that the cells that were induced by the peptide stimulation are not so-called CD8⁺ CTL, but rather CD4⁺ T-lymphocytes. In general, CD4⁺ T-lymphocytes are thought to recognize a complex of an MHC class II molecule and a peptide on an antigen-presenting cell and produce various cytokines, such as IFN-γ. However, given that production of IFN-γ was inhibited by the anti-class I antibody, as described above, and based on a recent report of a case in which CD4⁺ cells recognize antigens in a class I-restricted manner (*Cancer Research*, 1999, Volume 59, pp. 6230-6238), it is possible that the CD4⁺ cells that were induced in the present example recognize antigens in a class I-restricted manner. That is to say, it is suggested that the cells that have been induced by each of the peptides are CD4⁺ cells, and that these cells recognize each peptide presented on the HLA-A2 molecules that are present on the surface of T2 cells and produce IFN-γ. In addition, given that the aforementioned production of IFN-γ was inhibited by anti-class II antibodies, the following may be deduced: class II molecules are expressed on the CD4⁺ cells that have been induced, while the CD4 molecule is expressed on T2 cells; the CD4 molecule is known to bind to the β2 region of an MHC class II molecule, and the binding stabilizes the T-cell antigen receptor (TCR)/peptide/MHC complex (*Cancer Research*, 1999, Volume 59, pp. 6230-6238). Accordingly, it can be deduced that the binding of T2 cells to CD4⁺ cells via a class II molecule is involved in the enhancement of the stability of the binding of the TCR on CD4⁺ cells to the complex of the class I molecule on T2 cells and the peptide. Or, there is also the possibility that a peptide with a weak affinity binds to a class II molecule that is expressed on CD4⁺ cells, and this complex is recognized by a CD4⁺ cell in the vicinity.

The present example was performed based on the results shown in Example 6, that is, based on the fact that cells that were induced from the PBMC derived from the MS patient or the healthy subject by each of the peptides recognized T2 cells that were pulsed with the peptide, but did not exhibit cytotoxic activity. Therefore, although the PBMC that were derived from healthy subjects were used in the present example, it can be deduced that in the present example, using PBMC derived from healthy subjects would be the same as PBMC that were derived from MS patients.

### Example 8: Detection of peptide specific antibody in serum of multiple sclerosis patients

The presence of immunoglobulin E (IgE) and immunoglobulin G (IgG) that specifically recognize P102 (SEQ ID NO: 2), P103 (SEQ ID NO: 3), P104 (SEQ ID NO: 4), or P106 (SEQ ID NO: 6) in the serum of a multiple sclerosis patient, a brain tumor patient, and a healthy subject was measured by the enzyme-linked immunosorbent assay (ELISA).

Each aforementioned peptide (20 µg/well) was immobilized in a 96-well Nunc Covalink flat-bottomed plate (Fisher Scientific), using disuccinimidyl suberate (PIERCE), following the product instructions. This plate was blocked with Block Ace (Yukijirushi) and washed with 0.05% Tween20-PBS; a serum or blood plasma sample was diluted with 0.05% Tween20-Block Ace and added to the plate at 100 µl/well. After incubation for 2 hours at 37°C, the plate was washed with 0.05% Tween20-PBS and further incubated for 2 hours at 37°C with rabbit anti-human IgE antibody (ε chain-specific), anti-human IgG antibody (γ chain-specific) (DAKO), or anti-human IgG subclass-specific antibody (Zymed Laboratories), each of which were diluted to 1:1000. This plate was washed 9 times; 100 µl of horseradish peroxidase dextran polymer linked to goat anti-rabbit Ig antibody (EnVision, DAKO) diluted to 1:100, was added to each well and further incubated for 40 minutes at room temperature. After washing, 100 µl of tetramethylbenzene substrate solution (KPL) was added. Thereafter, the reaction was stopped by adding 1 M phosphoric acid, and the optical density (OD) at 450 nm was measured. In so doing, peptides derived from EBV, HIV, SART2 (JP-11-318455), or SART3 (*Cancer Research,* 1999, Volume 59, pp. 4956-4063) were used as controls and measured similarly. In addition, the OD value arising from a non-specific response obtained by using a peptide derived from HIV was subtracted from the aforementioned OD values. Furthermore, when the soluble peptide corresponding to the respective immobilized peptide was added to each antibody measurement system, the response was inhibited and the optical density decreased, but no inhibition was observed if an unrelated peptide was added. Therefore, it is revealed that the aforementioned IgE and IgG that were measured are specific antibodies against the respective peptides.

The results with respect to the sera of MS patients, brain tumor patients, and healthy subjects are shown in Fig. 14, Fig. 15, and Fig. 16, respectively. In each of the figures, Fig. A shows IgE and Fig. B shows IgG. Fig. 14A clearly reveals that IgE that specifically recognizes P102 (SEQ ID NO: 2) or P104 (SEQ ID NO: 4) is present in the serum of MS patients. However, IgG was not detected. Meanwhile, both IgE and IgG were not detected in the sera of brain tumor patients or healthy subjects.

So far, no report exists in the literature that suggests the involvement of antibodies in MS. This may be due to MS being predominantly a pathology of helper T1 cells. Meanwhile, the results from animal experiments that have been performed based on the analysis of such pathological mechanisms are in no way linked to the results from clinical experiments (Martin, R. *et al., Nature Immunology,* 2001, Volume 2, pp. 785-788). Therefore, in terms of MS, a mechanism that has not yet been elucidated may be involved in the pathology. In the present example, it has been shown that an IgE that recognizes a peptide pertaining to the present invention is present in the blood of the MS patient. Therefore, it is also possible that the stimulation of mast cells by the IgE that recognizes the aforementioned peptide is related to the pathological formation of MS. From the foregoing, prevention and/or treatment of MS is thought to be possible by using the peptide described above, for instance, by methods that induce immunological tolerance or the like.

### INDUSTRIAL APPLICABILITY

A gene coding for a tumor antigen that is recognized by HLA-A2-restricted tumor-specific cytotoxic T-lymphocytes was isolated from the cDNA library of the human glioma cell strain KNS60, using a gene expression cloning method, and identified. Furthermore, a peptide and a polypeptide having the epitope of the tumor antigen were found based on the tumor antigen coded by the gene that was obtained.

These peptide and polypeptide induced HLA-A2-restricted tumor-specific cytotoxic T-lymphocytes from peripheral blood mononuclear cells derived from a brain tumor patient, which recognize the peptide and the polypeptide and may damage tumor cells. The HLA-A2 allele is found in approximately 40% of Japanese, approximately 53% of Chinese, approximately 49% of North American Caucasians, approximately 38% of South American Caucasians, and approximately 23% of African Blacks. Therefore, according to the present invention, specific immunotherapy of cancers, for example, brain tumors and the like, is possible, and a considerable contribution to cancer therapy can be expected. In addition, the present invention also contributes considerably to fundamental research into molecules involved in recognition of cancer by cytotoxic T-lymphocytes.

Furthermore, it was discovered that the peripheral blood mononuclear cells, derived from the multiple sclerosis patient, which have been stimulated with the peptides of the present invention, recognize each corresponding peptide and enhance production of IFN-γ, but do not recognize the tumor cells; that the production of IFN-γ is due to CD4-positive cells; and that IgE that recognizes the peptide is present in the serum of multiple sclerosis patients. It is possible that the peptides of the present invention are involved in multiple sclerosis and may be utilized in the elucidation, as well as prevention and/or treatment, of multiple sclerosis.

### Sequence Listing Free Text

Sequence Listing SEQ ID NO: 1: designed peptide recognized by HLA-A2-restricted cytotoxic T-lymphocytes.
Sequence Listing SEQ ID NO: 2: designed peptide recognized by HLA-A2-restricted cytotoxic T-lymphocytes.
Sequence Listing SEQ ID NO: 3: designed peptide recognized by HLA-A2-restricted cytotoxic T-lymphocytes.
Sequence Listing SEQ ID NO: 4: designed peptide recognized by HLA-A2-restricted cytotoxic T-lymphocytes.
Sequence Listing SEQ ID NO: 5: designed peptide recognized by HLA-A2-restricted cytotoxic T-lymphocytes.
Sequence Listing SEQ ID NO: 6: designed peptide recognized by HLA-A2-restricted cytotoxic T-lymphocytes.
Sequence Listing SEQ ID NO: 7: designed peptide recognized by HLA-A2-restricted cytotoxic T-lymphocytes.
Sequence Listing SEQ ID NO: 8: designed peptide recognized by HLA-A2-restricted cytotoxic T-lymphocytes.
Sequence Listing SEQ ID NO: 9: designed peptide recognized by HLA-A2-restricted cytotoxic T-lymphocytes.
Sequence Listing SEQ ID NO: 10: designed peptide recognized by HLA-A2-restricted cytotoxic T-lymphocytes.
Sequence Listing SEQ ID NO: 11: designed peptide recognized by HLA-A2-restricted cytotoxic T-lymphocytes.
Sequence Listing SEQ ID NO: 12: designed peptide recognized by HLA-A2-restricted cytotoxic T-lymphocytes.
Sequence Listing SEQ ID NO: 13: designed peptide recognized by HLA-A2-restricted cytotoxic T-lymphocytes.

## Claims

1. A peptide having the amino acid sequence set forth in any one of SEQ ID NO: 1 to 13 in the Sequence Listing.

2. A polypeptide having the amino acid sequence set forth in any one of SEQ ID NO: 14 to 18 in the Sequence Listing.

3. A peptide having the amino acid sequence set forth in any one of SEQ ID NO: 1 to 13 in the Sequence Listing, wherein the peptide is recognized by a cytotoxic T-lymphocyte and/or induces a cytotoxic T-lymphocyte.

4. The peptide of claim 3, wherein being recognized by a cytotoxic T-lymphocyte and/or inducing a cytotoxic T-lymphocyte is being recognized by a cytotoxic T-lymphocyte in an HLA-A2-restricted manner and/or inducing a cytotoxic T-lymphocyte in an HLA-A2-restricted manner.

5. A polypeptide having the amino acid sequence set forth in any one of SEQ ID NO: 14 to 18 in the Sequence Listing, wherein the polypeptide is recognized by a cytotoxic T-lymphocyte and/or induces a cytotoxic T-lymphocyte.

6. The polypeptide of claim 5, wherein being recognized by a cytotoxic T-lymphocyte and/or inducing a cytotoxic T-lymphocyte, is being recognized by a cytotoxic T-lymphocyte in an HLA-A2-restricted manner and/or inducing a cytotoxic T-lymphocyte in an HLA-A2-restricted manner.

7. A medicament comprising one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

8. A cancer vaccine that contains one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

9. The cancer vaccine of claim 8, which is used in the treatment of a brain tumor.

10. An agent for inducing a cytotoxic T-lymphocyte that contains one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

11. A method for inducing a cytotoxic T-lymphocyte comprising using of one or more peptides selected from peptides having the respective amino acid sequences set forth in SEQ ID NO: 1 to 13 in the Sequence Listing, and/or one or more polypeptides selected from polypeptides having the respective amino acid sequences set forth in SEQ ID NO: 14 to 18 in the Sequence Listing.

12. A method for inducing a cytotoxic T-lymphocyte comprising the steps of:
i) incubating an antigen-presenting cell that retains HLA-A2 with a peptide having the amino acid sequence set forth in any one of SEQ ID NO: 1 to 13 in the Sequence Listing; or
ii) expressing a polypeptide having the amino acid sequence set forth in any one of SEQ ID NO: 14 to 18 in the Sequence Listing in an antigen-presenting cell that retains HLA-A2; and
iii) using the cell obtained in said step i) or said step ii) for stimulating a group of cells that contain a precursor cell of the cytotoxic T-lymphocyte.

13. The medicament of claim 7, which is used in the prevention and/or the treatment of multiple sclerosis.

14. A polynucleotide having a nucleotide sequence that codes for a peptide having the amino acid sequence set forth in any one of SEQ ID NO: 1 to 13 in the Sequence Listing or a polypeptide having the amino acid sequence set forth in any one of SEQ ID NO: 14 to 18 in the Sequence Listing, or a complementary nucleotide sequence thereof.

15. A polynucleotide having the nucleotide sequence set forth in any one of SEQ ID NO: 19 to 23 in the Sequence Listing, or a complementary nucleotide sequence thereof.

16. A polynucleotide having the nucleotide sequence set forth in any one of SEQ ID NO: 19 to 23 in the Sequence Listing, wherein the nucleotide sequence is such that the polypeptide coded by said nucleotide sequence is recognized by a cytotoxic T-lymphocyte, and/or induces a cytotoxic T-lymphocyte, or a complementary nucleotide sequence thereof.

17. The polynucleotide of claim 11, wherein being recognized by a cytotoxic T-lymphocyte and/or inducing a cytotoxic T-lymphocyte is being recognized by a cytotoxic T-lymphocyte in an HLA-A2-restricted manner and/or inducing a cytotoxic T cell in an HLA-A2-restricted manner.

18. A polynucleotide that hybridizes under stringent conditions with the polynucleotide according to any one of claims 14 to 17.

19. A recombinant vector comprising the polynucleotide according to any one of claims 14 to 18.

20. The recombinant vector of claim 19, wherein said recombinant vector is a recombinant expression vector.

21. A transformant that has been transformed by the recombinant vector of claim 19 or claim 20.

22. A method for preparing the peptide of claim 1, claim 3 or claim 4 or the polypeptide of claim 2, claim 5 or claim 6, wherein the method comprises a step of culturing a transformant that has been transformed by the recombinant vector of claim 20.

23. An antibody that immunologically recognizes the peptide of claim 1, claim 3 or claim 4 and/or the polypeptide of claim 2, claim 5 or claim 6.

24. A method for identifying: a compound that interacts with the peptide of claim 4 or the polypeptide of claim 6 and/or an HLA-A2 molecule, and at least enhances the recognition of said peptide or said polypeptide by an HLA-A2-restricted cytotoxic T-lymphocyte; and/or a compound that interacts with the polynucleotide according to any one of claims 14 to 18 and enhances the expression thereof, wherein the method comprises using at least one of: the peptide of claim 4; the polypeptide of claim 6; the polynucleotide according to any one of claims 14 to 18; the recombinant vector of claim 19 or claim 20; the transformant of claim 21; or the antibody of claim 23.

25. A compound that is identified by the method of claim 24.

26. A compound that enhances the recognition of at least one of the peptide of claim 4 or the polypeptide of claim 6 by an HLA-A2-restricted cytotoxic T-lymphocyte.

27. A compound that interacts with the polynucleotide according to any one of claims 14 to 18 and enhances the expression thereof.

28. A pharmaceutical composition for use in cancer therapy that contains an effective dose of at least one of: the peptide of claim 1, claim 3 or claim 4; the polypeptide of claim 2, claim 5 or claim 6; the polynucleotide according to any one of claims 14 to 18; the recombinant vector of claim 19 or claim 20; the transformant of claim 21; the antibody of claim 23; and the compound according to any one of claims 25 to 27.

29. A pharmaceutical composition for use in the prevention and/or treatment of multiple sclerosis that contains an effective dose of at least one of: the peptide of claim 1, claim 3 or claim 4; the polypeptide of claim 2, claim 5 or claim 6; the polynucleotide according to any one of claims 14 to 18; the recombinant vector of claim 19 or claim 20; the transformant of claim 21; the antibody of claim 23; and the compound according to any one of claims 25 to 27.

30. A method for quantitatively and/or qualitatively measuring: the peptide of claim 1, claim 3 or claim 4; the polypeptide of claim 2, claim 5 or claim 6; or the polynucleotide according to any one of claims 14 to 18.

31. The method of claim 30, which is used in cancer screening.

32. A reagent kit comprising at least one of: the peptide of claim 1, claim 3 or claim 4; the polypeptide of claim 2, claim 5 or claim 6; the polynucleotide according to any one of claims 14 to 18; and the antibody of claim 23.

33. A reagent kit for use in the method of claim 24, claim 30 or claim 31, wherein the reagent kit comprises at least one of: the peptide of claim 1, claim 3 or claim 4; the polypeptide of claim 2, claim 5 or claim 6; the polynucleotide according to any one of claims 14 to 18; and the antibody of claim 23.
